(19) Europäisches Patentamt
European Patent Office
Office européen des brevets

(11) Veröffentlichungsnummer: **0 149 974**
**B1**

(12) # EUROPÄISCHE PATENTSCHRIFT

(45) Veröffentlichungstag der Patentschrift: **21.09.88**

(51) Int. Cl.[4]: **A 01 N 25/32**, C 07 D 265/28, C 07 D 239/16

(21) Anmeldenummer: **84810599.5**

(22) Anmeldetag: **06.12.84**

(54) **Mittel zum Schützen von Kulturpflanzen vor der phytotoxischen Wirkung von Herbiziden.**

(30) Priorität: **12.12.83 US 560465**
**12.12.83 US 560466**

(43) Veröffentlichungstag der Anmeldung:
**31.07.85 Patentblatt 85/31**

(45) Bekanntmachung des Hinweises auf die Patenterteilung:
**21.09.88 Patentblatt 88/38**

(84) Benannte Vertragsstaaten:
**AT BE CH DE FR GB IT LI NL**

(56) Entgegenhaltungen:
**EP-A-0 006 540**
**DE-A-1 595 863**
**DE-A-2 620 101**

**JOURNAL OF MEDICINAL CHEMISTRY, Band 12, Nr. 2, März 1969, Seiten 290-294, Washington, D.C., US; R.N. PRASAD: "Potential antihypertensive agents. III. 3,4-Dihydro-2H-1,4-benzothiazine derivatives"**
**J. Org. Chem., Band 48, 1983, Seiten 4082-4087**

(73) Patentinhaber: **CIBA- GEIGY AG, Klybeckstrasse 141, CH- 4002 Basel (CH)**

(72) Erfinder: **Moser, Hans, Dr., Blumenrain 3, CH- 4312 Magden (CH)**

**EP 0 149 974 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein Mittel zum Schutz von Kulturpflanzen vor der phytotoxischen Wirkung von Herbiziden, welches als Herbizid-antagonisierenden Wirkstoff ein Acylamid-Derivat enthält, ferner Mittel welche neben diesem antagonisierenden Wirkstoff bereits das Herbizid enthalten und ein Verfahren zur selektiven Unkrautbekämpfung mittels eines Herbizides und diesem Gegenmittel. Die Erfindung umfasst auch neue Acylamid-Derivate und deren Herstellung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäuren usw. bei der Anwendung in wirksamer Dosis gelegentlich neben den zu bekämpfenden Unkräutern auch die Kulturpflanzen in gewissem Masse schädigen. Überdosen werden oft ungewollt und zufälligerweise appliziert, wenn sich Randzonen beim streifenweisen Spritzen überdecken, sei es durch Windeinwirkung der durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Überdosis wirkt. Die Qualität des Saatgutes kann bei der Herbizidverträglichkeit auch eine Rolle spielen. Umdiesem Problem zubegegnen, sind schon verschiedene Stoffe vorgeschlagen worden, welche befähigt sind, die schädigende Wirkung des Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

So beschreibt die Britische Patentschrift 1 277 557 die Behandlung von Samen bzw. Sprösslingen von Weizen und Sorghum mit gewissen Oxamsäureestern und Amiden zum Schutz vor dem Angriff durch "ALACHLOR" (N-Methoxymethyl-N-chloracetyl-2,6-diäthylanilin). In den Deutschen Offenlegungsschriften 1 952 910 und 2 245 471, sowie in der Französischen Patentschrift 2 021 611 werden Gegenmittel zur Behandlung von Getreide-, Mais- und Reissamen zum Schutz gegen die schädigende Einwirkung von herbizid wirksamen Thiolcarbamaten vorgeschlagen. Gemäss der Deutschen Patentschrift 1 567 075 und der US-Patentschrift 3 131 509 werden Hydroxyaminoacetanilide und Hydantoine für den Schutz von Getreidesamen gegenüber Carbamaten verwendet.

Die direkte pre- oder postemergente Behandlung gewisser Nutzpflanzen mit Gegenmitteln als Antagonisten bestimmter Herbizidklassen auf einer Anbaufläche ist in den Deutschen Offenlegungsschriften 2 141 586 und 2 218 097, sowie im US-Patent 3 867 444 beschrieben.

Ferner können Maispflanzen gemäss der deutschen Offenlegungsschrift 2 402 983 wirksam vor Schädigung durch Chloracetanilide geschützt werden, indem man dem Boden als Gegenmittel ein N-disubstituiertes Dichloracetamid zuführt. Derartige Verbindungen werden gemäss US-PS-4 137 070 auch als Antidotes bei herbiziden Thiocarbamaten verwendet oder gemäss DE-OS-2 828 265 und 2 828 293 als Antidotes gegen herbizide Acetanilide.

Es wurde nun gefunden, dass sich überraschenderweise eine Gruppe von Acylamid-Derivaten hervorragend dazu eignet, Kulturpflanzen gegen schädigende Wirkung von Agrarchemikalien, wie beispielsweise Pflanzenschutzmitteln, insbesondere Herbiziden, zu schützen. Diese Acylamid-Derivate werden daher im folgenden auch äls "Gegenmittel", "Antidot" oder "Safener" bezeichnet.

Acylamid-Derivate, die zum Schützen von Kulturpflanzen gegen die schädigende Wirkung von Agrarchemikalien geeignet sind entsprechen der Formel I

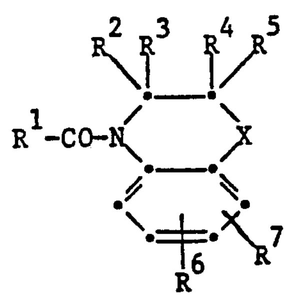

(I) ,

worin

X Sauerstoff, Schwefel, -SO- oder $-SO_2-$,

$R^1$ $C_1$-$C_6$-Halogenalkyl, $C_1$-$C_6$-Cyanalkyl oder $C_2$-$C_6$-Halogenalkenyl,

$R^2$ und $R^5$ unabhängig voneinander Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R^3$ und $R^4$ unabhängig voneinander Wasserstoff, $C_2$-$C_4$-Alkenyl, $C_2$-$C_4$-Alkinyl, Cyan, $C_2$-$C_4$-Alkoxyalkyl, $-COOR^8$, $-CO-NR^9R^{10}$ oder unsubstituiertes oder durch Halogen, Cyan oder -CO-A substituiertes $C_1$-$C_4$-Alkyl,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl oder $C_1$-$C_4$-Halogen-alkoxy,

A $C_1$-$C_4$-Alkyl, $C_2$-$C_4$-Alkenyl, $C_1$-$C_4$-Alkoxy, $C_3$-$C_4$-Alkenyloxy, $C_3$-$C_4$-Alkinyloxy oder $-NR^{11}R^{12}$,

$R^8$, $R^{10}$ und $R^{12}$ unabhängig voneinander Wasserstoff, $C_1$-$C_4$-Alkyl, $C_3$-$C_4$-Alkenyl, $C_3$-$C_4$-Alkinyl oder $C_3$-$C_4$-Alkoxyalkyl und

$R^9$ und $R^{11}$ unabhängig voneinander Wasserstoff, $C_1$-$C_4$-Alkyl, $C_3$-$C_4$-Alkenyl, $C_1$-$C_4$-Alkoxy, $C_3$-$C_4$-Alkinyl oder $C_3$-$C_4$-Alkoxyalkyl bedeuten, wobei auch

$R^9$ and $R^{10}$ sowie $R^{11}$ und $R^{12}$ zusammen mit dem sie bindenden Stickstoffatom einen 5- bis 6-gliedrigen gesättigten Heterocyclus bilden können, der als Ringglied gegebenenfalls ein Sauerstoff- oder Schwefelatom oder eine -NH- oder -N($C_1$-$C_4$-Alkyl)-Brücke enthalten kann.

Soweit optisch isomere Verbindungen der Formel I existieren, sind im Rahmen der vorliegenden Erfindung sowohl die optisch reinen Isomere wie auch die Isomerengemische zu verstehen.

Unter Halogen selbst in den Definitionen sowie Halogen als Teil in Halogenalkoxy, Halogenalkyl oder Halogenalkenyl sind Fluor, Chlor, Brom und Jod, vorzugsweise jedoch Fluor, Chlor und Brom, insbesondere aber Chlor zu verstehen.

In den Definitionen ist unter Alkyl geradkettiges oder verzweigtes Alkyl zu verstehen; z. B.: Methyl, Äthyl, n-Propyl, i-Propyl oder die vier isomeren Butyl.

Unter Alkoxy ist zu verstehen: Methoxy, Äthoxy n-Propyloxy i-Propyloxy oder die vier isomeren Butyloxyreste, insbesondere aber Methoxy, Äthoxy oder i-Propyloxy.

Beispiele für ungesättigte Substituenten oder Substituententeile sind Vinyl, Vinyloxy, Allyl, Allyloxy, Propargyl, Propargyloxy, Methallyl, Methallyloxy, Butenyl, Butenyloxy, Butinyl, Butinyloxy, Chlorvinyl, Dichlorvinyl, Trichlorvinyl, 3,3,3-Trifluor-1-propenyl, 3,3,3-Trichlor-1-propenyl oder 2,3-Dichlorpropargyl.

Alkoxyalkylreste werden repräsentiert durch Methoxymethyl, Äthoxymethyl, Methoxyäthyl und Äthoxyäthyl, insbesondere aber Methoxyäthyl. Halogenalkyl als Substituent selbst oder als Teil eines anderen Substituenten wie Halogenalkoxy oder Halogenalkylthio steht in der Regel für Chlormethyl, Fluormethyl, Difluormethyl, Trifluormethyl, Dichlormethyl, Trichlormethyl 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 2-Chlorpropyl, 1,1,2,3,3,3-Hexafluorpropyl, 3,3,3-Trichlorpropyl, 2,2,2-Trichloräthyl, 1-Chloräthyl, insbesondere aber Chlormethyl, Dichlormethyl, Trichlormethyl und 1-Chloräthyl.

Wegen ihrer Wirkung als Herbizidantagonisten sind solche hervorzuheben, in denen entweder

a) X für Sauerstoff steht, oder
b) $R^1$ für $C_1$-$C_6$-Halogenalkyl oder $C_2$-$C_6$-Halogenalkenyl steht, oder
c) $R^2$ und $R^5$ für Wasserstoff stehen, oder
d) $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl bedeuten, oder
e) $R^6$ und $R^7$ unabhängig voneinander Wasserstoff oder $C_1$-$C_4$-Alkyl bedeuten.

Von den Wirkstoffen der Untergruppe b sind diejenigen bevorzugt, in denen $R^1$ für $C_1$-$C_4$-Halogenalkyl, insbesondere Dichlormethyl, steht, und von der Untergruppe d diejenigen, in denen $R^3$ und $R^4$ unabhängig voneinander Wasserstoff oder Methyl bedeuten.

Eine ganz besonders hervorzuhebende Gruppe von Wirkstoffen ist jene, in welchen X Sauerstoff, $R^1$ $C_1$-$C_6$-Halogenalkyl oder $C_2$-$C_6$-Halogenalkenyl, $R^2$ und $R^5$ Wasserstoff, $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl und $R^6$ und $R^7$ unabhängig voneinander Wasserstoff oder $C_1$-$C_4$-Alkyl bedeuten.

Hiervon sind widerum die Wirkstoffe bevorzugt, in denen $R^1$ für $C_1$-$C_4$-Halogenalkyl und $R^3$, $R^4$, $R^6$ und $R^7$ unabhängig voneinander für Wasserstoff oder Methyl stehen. Darunter sind die Wirkstoffe hervorzuheben, in denen $R^2$ für Dichlormethyl steht.

Als bevorzugte Einzelwirkstoffe sind zu nennen: 4-Dichloracetyl-2,3-dihydro-3,6-dimethyl-1,4-benzoxazin, 4-Dichloracetyl-2,3-dihydro-3-methyl-1,4-benzoxazin oder 4-(2-Chlorpropionyl)-2,3-dihydro-3-methyl-1,4-benzoxazin.

Die meisten Verbindungen der Formel I sind neu. Manche sind in der Literatur beschrieben. So sind einzelne Halogenalkanoylamide pharmazeutische Wirkstoffe oder als Zwischenprodukte für pharmazeutisch wirksame Substanzen aus dem britischen Patent 1 137 796 (Zwischenprodukte für Analgetika), J. Med. Chemistry 290 - 294, 1969 (Antihypertonika, C.A. 70, 106447m, 1969), DE-OS-2 656 806 (Antidepressiva), der japanischen Patentpublikation 81 029 676 (Anticonvulsiva, C.A. 95, 80985z, 1981) und J. Org. Chem. 1983, Bd. 48, 4082 - 4087 (ohne Wirkungsangabe) bekannt.

Neu sind die Verbindungen der Formel I

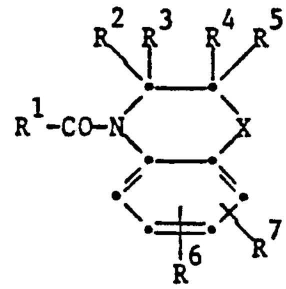

(I) ,

worin

X Sauerstoff, Schwefel, -SO- oder $-SO_2-$,

$R^1$ $C_1$-$C_6$-Cyanalkyl, $C_2$-$C_6$-Halogenalkenyl oder durch mindestens zwei Halogenatome substituiertes $C_1$-$C_6$-Alkyl,

$R^2$ und $R^5$ unabhängig voneinander Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R^3$ und $R^4$ unabhängig voneinander Wasserstoff, $C_2$-$C_4$-Alkenyl, $C_2$-$C_4$-Alkinyl, Cyan, $C_2$-$C_4$-Alkoxyalkyl, $-COOR^8$, $-CO-NR^9R^{10}$ oder unsubstituiertes oder durch Halogen, Cyan oder -CO-A substituierter $C_1$-$C_4$-Alkyl,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl oder $C_1$-$C_4$-Halogenalkoxy,

A $C_1$-$C_4$-Alkyl $C_2$-$C_4$-Alkenyl, $C_1$-$C_4$-Alkoxy, $C_3$-$C_4$-Alkenyloxy, $C_3$-$C_4$-Alkinyloxy oder $-NR^{11}R^{12}$,

$R^8$, $R^{10}$ und $R^{12}$ unabhängig voneinander Wasserstoff, $C_1$-$C_4$-Alkyl, $C_3$-$C_4$-Alkenyl, $C_3$-$C_4$-Alkinyl oder $C_3$-$C_4$-Alkoxyalkyl und

$R^9$ und $R^{11}$ unabhängig voneinander Wasserstoff, $C_1$-$C_4$-Alkyl, $C_3$-$C_4$-Alkenyl, $C_1$-$C_4$-Alkoxy, $C_3$-$C_4$-Alkinyl oder $C_3$-$C_4$-Alkoxyalkyl bedeuten, wobei auch

$R^9$ and $R^{10}$ sowie $R^{11}$ und $R^{12}$ zusammen mit dem sie bindenden Stickstoffatom einen 5- bis 6-gliedrigen gesättigten Heterocyclus bilden können, der als Ringglied gegebenenfalls ein Sauerstoff- oder Schwefelatom oder eine -NH- oder -N($C_1$-$C_4$-Alkyl)-Brücke enthalten kann, unter Ausnahme der Verbindung 4-Trifluoracetyl-2,3-dihydro-1,4-benzothiazin.

Im Hinblick auf ihren Safener-Effekt hervorzuhebende neue Verbindungen sind solche, in denen entweder

a) X für Sauerstoff steht, oder

b) $R^1$ für $C_2$-$C_6$-Halogenalkenyl oder durch mindestens zwei Halogenatome substituiertes $C_1$-$C_6$-Alkyl steht, oder

c) $R^2$ und $R^5$ Wasserstoff bedeuten, oder

d) $R^3$ und $R^4$ unabhängig voneinander Wasserstoff, $C_1$-$C_4$-Alkyl $C_1$-$C_4$-Alkoxycarbonylmethyl oder $C_1$-$C_4$-Alkoxycarbonyl bedeuten oder

e) $R^6$ und $R^7$ unabhängig voneinander Wasserstoff oder $C_1$-$C_4$-Alkyl bedeuten.

Von den neuen Verbindungen sind aus der Gruppe b solche hervorzuheben, in denen $R^1$ für durch mindestens zwei Halogenatome substituiertes $C_1$-$C_4$-Alkyl, insbesondere Dichlormethyl, steht, und aus der Gruppe d solche, in denen $R^3$ und $R^4$ unabhängig voneinander Wasserstoff oder Methyl bedeuten.

Eine besonders bevorzugte Gruppe von neuen Verbindungen der Formel I wird dadurch gebildet, dass X Sauerstoff, $R^1$ $C_2$-$C_6$-Halogenalkenyl, oder durch mindestens zwei Halogenatome substituiertes $C_1$-$C_4$-Alkyl, $R^2$ und $R^5$ Wasserstoff, $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl und $R^6$ und $R^7$ unabhängig voneinander Wasserstoff oder $C_1$-$C_4$-Alkyl bedeuten.

Eine weitere herausragende Gruppe bilden hiervon diejenigen neuen Verbindungen, in denen $R^1$ für durch mindestens zwei Halogenatome substituiertes $C_1$-$C_4$-Alkyl und $R^3$, $R^4$, $R^6$ und $R^7$ unabhängig voneinander für Wasserstoff oder Methyl stehen. Darunter sind die Verbindungen hervorzuheben, in denen $R^1$ für Dichlormethyl steht.

Als bevorzugte neue Einzelverbindungen sind zu nennen: 4-Dichloracetyl-2,3-dihydro-3,6-dimethyl-1,4-benzoxazin, 4-Dichloracetyl-2,3-dihydro-3-methyl-1,4-benzoxazin und 4-(2-Chlorpropionyl)-2,3-dihydro-3-methyl-1,4-benzoxazin.

Diese neuen Verbindungen werden hergestellt, indem man ein Acylhalogenid der Formel II

$R^1$ - CO- Hal (II),

worin $R^1$ $C_1$-$C_6$-Cyanalkyl, $C_2$-$C_6$-Halogenalkenyl oder durch mindestens zwei Halogenatome substituiertes $C_1$-$C_6$-Alkyl und Hal Chlor oder Brom bedeuten, in Gegenwart eines säurebindenden Mittels mit einem Amin der Formel III

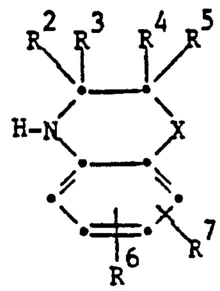

(III),

worin $R^2$, $R^3$, $R^4$, $R^5$ $R^6$, $R^7$ und X die unter Formel I gegebene Bedeutung haben umsetzt, mit der Massgabe, dass $R^1$ nicht für Trifluormethyl stehen darf, wenn gleichzeitig $R^2$, $R^3$, $R^4$, $R^5$ $R^6$ und $R^7$ Wasserstoff und X Schwefel bedeuten.

Die Reaktion wird zweckmässigerweise in einem reaktionsinerten Lösungsmittel bei Normaldruck durchgeführt. Als Lösungsmittel eignen sich beispielsweise aliphatische oder aromatische Kohlenwasserstoffe wie Benzol, Toluol, Xylole, Cyclohexan, Petroläther, halogenierte Kohlenwasserstoffe wie Chlorbenzol, Methylenchlorid, Äthylenchlorid, Chloroform, Äther und ätherartige Verbindungen wie Diäthyläther, Diisopropyläther, t-Butylmethyläther, Dimethoxyäthan, Dioxan, Tetrahydrofuran, Anisol; Ketone wie Aceton, Methyläthylketon; Ester wie Äthylacetat, Butylacetat und Gemische solcher Lösungsmittel untereinander.

Als säurebindende Mittel sind insbesondere tertiäre Amine wie Trimethylamin, Triäthylamin, Chinuclidin, 1,4-Diazabicyclo-[2.2.2]-octan, 1,5-Diazabicyclo[4.3.0]non-5-en oder 1,8-Diazabicyclo[5.4.0]undec-7-en geeignet. Es können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die ausgangsverbindungen der Formeln II und III sind allgemein bekannt und können nach an sich bekannten Methoden hergestellt werden.

Ein Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die preemergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1 : 100 bis 5 : 1. In der Regel wird bei einem Mengenverhältnis von Gegenmittel zu Herbizid von 1 : 5 bis 1 : 50 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1 - 10 g Gegenmittel benötigt. In der Regel wird mit 0,1-5 g Gegenmittel pro kg Samen bereits die volle Schutzwirkung erreicht. Falls das Gegenmittel kurz vor der Aussaat durch Samenquellung appliziert werden soll, so werden zweckmässig Lösungen des Gegenmittels verwendet, welche den Wirkstoff in einer Konzentration von 1 - 10'000 ppm enthalten. In der Regel wird mit Konzentrationen des Gegenmittels von 100 - 1'000 ppm die volle Schutzwirkung erreicht.

In der Regel liegt zwischen Protektiven Massnahmen, wie Samenbeizung und Behandlung von Stecklingen mit einem Gegenmittel der Formel I und der möglichen späteren Feldbehandlung mit Agrarchemikalien ein längerer Zeitraum. Vorbehandeltes Saat- und Pflanzengut kann später in der Landwirtschaft, im Gartenbau und in der Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Öle, Zucker, Stärke, Eiweiss usw., produzieren und zu diesem Zweck angebaut werden. Zu diesen Pflanzen gehören beispielsweise sämtliche Getreidearten, wie Weizen, Roggen, Gerste und Hafer, daneben vor allem Reis, Kulturhirse, Mais, Baumwolle, Zuckerrüben, Zuckerrohr, Soja, Bohnen und Erbsen.

Das Gegenmittel kann überall dort eingesetzt werden wo eine Kulturpflanze der vorgenannten Art vor der schädlichen Wirkung einer Agrarchemikalie geschützt werden soll. Dabei kommen als Agrarchemikalien in erster Linie Herbizide der verschiedensten Stoffklassen, insbesondere jedoch Halogenacetanilide und Thiocarbamate, in Betracht.

Halogenacetanilide, deren schädigende Wirkung gegenüber Kulturpflanzen mit Hilfe der Acylamid-Derivate der Formel I aufgehoben werden kann, sind bereits in grosser Zahl bekannt geworden. Solche Halogenacetaniliden können durch die folgende allgemeine Formel IV beschrieben werden:

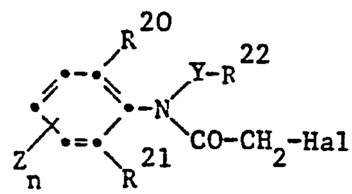

(IV)

In dieser Formel bedeuten Hal Halogen, insbesondere Chlor oder Brom, $R^{20}$ und $R^{21}$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die vorgenannten Reste Z vorzugsweise in 3-Stellung in Bezug auf das Stickstoffatom stehen, n 0 bis 3, Y Alkylen, insbesondere Methylen, 1,1- und 1,2-Äthylen, wobei 1,2-Äthylen durch 1 - 2 niedere Alkylgruppen substituiert sein kann, und $R^{22}$ niederes Alkoxy, Hydroxycarbonyl, Alkoxycarbonyl, Carbamoyl, N-Alkylcarbamoyl, N,N-Dialkylcarbamoyl, Cyano, ein gegebenenfalls substituierter stickstoffhaltiger heterocyclischer Rest, Alkanoyl, gegebenenfalls substituiertes Benzoyl, gegebenenfalls substituiertes 1,3,4-Oxadiazol-2-yl, 1,3,4-Thiadiazol-2-yl, 1,3,4-Triazol-3-yl oder 1,3,4-Triazol-1-yl bedeutet.

Als einzelne Vertreter solcher Halogenacetanilide seien beispielsweise die folgenden genannt:

N-Äthoxymethyl-N-chloracetyl-2-äthyl-6-methylanilin,
N-Chloracetyl-N-methoxymethyl-2,6-diäthylanilin,
N-Chloracetyl-N-(2-methoxyäthyl)-2,6-dimethylanilin,
N-(2-Allyloxyäthyl)-N-chloracetyl-2,6-dimethylanilin,
N-Chloracetyl-N-(2-n-propoxyäthyl)-2,6-dimethylanilin,
N-Chloracetyl-N-(2-isopropoyxäthyl)-2,6-dimethylanilin,
N-Chloracetyl-N-(2-methoxyäthyl)-2-äthyl-6-methylanilin,
N-Chloracetyl-N-(methoxyäthyl)-2,6-diäthylanilin,
N-(2-Äthoxyäthyl)-N-chloracetyl-2-äthyl-6-methylanilin,
N-Chloracetyl-N-(2-methoxy-1-methyläthyl)-2-methylanilin
N-Chloracetyl-N-(2-methoxy-1-methyläthyl)-2,6-dimethylanilin
N-Chloracetyl-N-(2-methoxy-1-methyläthyl)-2,6-diäthylanilin
N-Chloracetyl-N-(2-methoxy-1-methyläthyl)-2-äthyl-6-methylanilin,
N-(2-Äthoxyäthyl)-N-chloracetyl-2,6-diäthylanilin,
N-Chloracetyl-N-(2-n-propoxyäthyl)-2-äthyl-6-methylanilin,
N-Chloracetyl-N-(2-n-propoxyäthyl)-2,6-diäthylanilin,
N-Chloracetyl-N-(2-isopropoxyäthyl)-2-äthyl-6-methylanilin,
N-Äthoxycarbonylmethyl-N-chloracetyl-2,6-dimethylanilin,
N-Äthoxycarbonylmethyl-N-chloracetyl-2,6-diäthylanilin,
N-Chloracetyl-N-methoxycarbonylmethyl-2,6-dimethylanilin,
N-Chloracetyl-N-(2,2-diäthoxyäthyl)-2,6-dimethylanilin,
N-Chloracetyl-N-(2-methoxy-1-methyläthyl)-2,3-dimethylanilin,
N-(2-Äthoxyäthyl)-N-chloracetyl-2-methylanilin,
N-Chloracetyl-N-(2-methoxyäthyl)-2-methylanilin,
N-Chloracetyl-N-(2-methoxy-2-methyläthyl)-2,6-dimethylanilin,
N-(2-Äthoxy-2-methyläthyl)-N-chloracetyl-2-äthyl-6-methylinilin,
N-Chloracetyl-N-(1-äthyl-2-methoxyäthyl)-2,6-dimethylanilin,
N-Chloracetyl-N-(2-methoxyäthyl)-2-methoxy-6-methylanilin,
N-n-Butoxymethyl-N-chloracetyl-2-tert.-butylanilin,
N-(2-Äthoxyäthyl-1-methyläthyl)-2,6-dimethylanilin,
N-Chloracetyl-N-(-2-methoxyäthyl)-2-chlor-6-methylanilin,
N-(2-Äthoxyäthyl)-N-chloracetyl-2-chlor-6-methylanilin,
N-(2-Äthoxyäthyl)-N-chloracetyl-2,3,6-trimethylanilin,
N-Chloracetyl-1-(2-methoxyäthyl)-2,3,6-trimethylanilin,
N-Chloracetyl-N-cyanomethyl-2,6-dimethylanilin,
N-But-3-in-1-yl-N-chloracetylanilin,
N-Chloracetyl-N-propargyl-2-äthyl-6-methylanilin,
N-Chloracetyl-N-(1,3-dioxolan-2-ylmethyl)-2,6-dimethylanilin;
N-Chloracetyl-N-(1,3-dioxolan-2-ylmethyl)-2-äthyl-6-methylanilin,
N-Chloracetyl-N-(1,3-dioxan-2-ylmethyl)-2-äthyl-6-methylanilin,
N-Chloracetyl-N-(2-furanylmethyl)-2,6-dimethylanilin,

N-Chloracetyl-N-(2-furanylmethyl)-2-äthyl-6-methylanilin,
N-Chloracetyl-N-(2-tetrahydrofuranylmethyl)-2,6-dimethylanilin,
N-Chloracetyl-N-(N-propargylcarbamoylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diäthylanilin,
N-(2-n-Butoxyäthyl)-N-chloracetyl-2,6-diäthylanilin,
N-Chloracetyl-N-(2-methoxy-1,2-dimethyläthyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-äthyl-6-methylanilin,

N-Chloracetyl-N-(1H-1,2,4-triazol-1-ylmethyl)-2,6-dimethylanilin
N-Chloracetyl-N-(1H-1,2,4-triazol-1-ylmethyl)-2 6-diäthylanilin,
N-Benzoylmethyl-N-chloracetyl-2,6-dimethylanilin,
N-Benzoylmethyl-N-chloracetyl-2-äthyl-6-methylanilin,
N-Chloracetyl-N-(5-methyl-1,3,4-oxadiazol-2-yl)-2,6-diäthylanilin
N-Chloracetyl-N-(5-methyl-1,3,4-oxadiazol-2-yl)-2-äthyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und
N-Chloracetyl-N-(1-methyl-5-methylthio-1,3,4-triazol-2-ylmethyl)-2,6-diäthylanilin.

Weitere Halogenacetanilide deren schädigende Wirkung auf Kulturpflanzen durch die neuen Acylamid-Derivate der Formel I aufgehoben werden kann, sind in R. Wegler, Chemie der Pflanzenschutz- und Schädlings bekämpfungsmittel, Bd. 8, Seiten 90 - 93 und Seiten 322 - 327 aufgeführt.

Herbizid wirksame Thiolcarbamate, vor deren phytotoxischen Wirkung Kulturpflanzen durch die neuen Acylamid-Derivate der Formel I geschützt werden können, sind ebenfalls bereits in grosser Zahl bekannt geworden. Die Schützwirkung der neuen Acylamid-Derivate der Formel I lässt sich insbesondere beim Einsatz von Thiolcarbamaten in Getreide, Reis oder veredelter Sorghum-Hirse vorteilhaft ausnützen.

Die Thiolcarbamate, vor deren phytotoxischer Wirkung Kulturpflanzen wie Getreide Reis und veredelte Sorghum-Hirse bevorzugt geschützt werden können, entsprechen den allgemeinen Formeln V und VI:

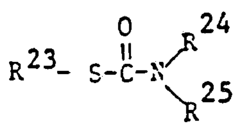 (V) und 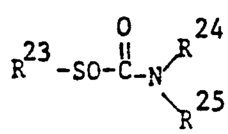 (VI)

In diesen Formeln bedeutet $R^{23}$ niederes Alkyl, Alkenyl, Chlorallyl, Dichlorallyl, Trichlorallyl, Benzyl oder 4-Chlorbenzyl, $R^{24}$ $C_2$-$C_4$-Alkyl und $R^{25}$ $C_2$-$C_4$-Alkyl oder Cyclohexyl, wobei die Reste $R^{24}$ und $R^{25}$ zusammen mit dem Stickstoffatom, an das sie gebunden sind, einen Hexahydro-1H-azepin-, Dekahydrochinolin- oder 2-Methyldekahydrochinolin-Ring bilden können.

Als einzelne Vertreter solcher Thiolcarbamate seien beispielsweise die folgenden genannt:

S-Äthyl-N,N-dipropylthiocarbamat,
S-Äthyl-N,N-diisobutylthiocarbamat,
S-2,3-Dichlorallyl-N,N-diisopropylthiolcarbamat,
S-Propyl-N-butyl-N-äthylthiolcarbamat,
S-2,3,3-Trichlorallyl-N,N-diisopropylthiocarbamat,
S-Propyl-N,N-dipropylthiolcarbamat,
S-Äthyl-N-äthyl-N-cyclohexylthiolcarbamat,
S-Äthyl-N-hexahydro-1H-azepin-1-carbothioat,
S-Isopropyl-N,N-hexamethylen-thiolcarbamat,
S-(p-Chlorbenzyl)-N,N-diäthylthiolcarbamat,
N-Äthylthiocarbonyl-cis-decahydrochinolin,
N-Propylthiocarbonyl-decahydrochinaldin,
S-Äthyl-N,N-bis(n-butyl)-thiolcarbamat und
S-tert.Butyl-N,N-bis(n-propyl)-thiolcarbamat.

Neben den Chloracetanilid- und Thiolcarbamaten kommen auch Herbizide anderer Stoffklassen in Betracht, wie beispielsweise

**Triazine und Triazinone:** 2,4-Bis(isopropylamino)-6-methylthio-1,3,5-triazin ("Prometryn"), 2,4-bis(äthylamino)-6-methylthio-1,3,5-triazin ("Simetryn"), 2-(1,2-Dimethylpropylamino)-4-äthylamino-6-methylthio-1,3,5-triazin ("Dimethametryn"), 4-Amino-6-tert.butyl-4,5-dihydro-3-methylthio-1,2,4-triazin-5-on ("Metribuzin"), 2-Chlor-4-äthylamino-6-isopropylamino-1,3,5-triazin ("Atrazin"), 2-Chlor-4,6-bis-(äthylamino)-1,3,5-triazin ("Simazin"), 2-tert.Butylamino-4-chlor-6-äthylamino-1,3,5-triazin ("Terbuthylazin"), 2-tert.Butylamino-4-äthylamino-6-methoxy-1,3,5-triazin ("Terbumeton"), 2-tert.Butylamino-4-äthylamino-6-methylthio-1,3,5-triazin ("Terbutryn"), 2-Äthylamino-4-isopropylamino-6-methylthio-1,3,5-triazin ("Ametryn"); 3,4-Bis-(methylamino)-6-tert.butyl-4,5-dihydro-1,2,4-triazin-5-on.

**Harnstoffe:** 1-(Benzothiazol-2-yl)-1,3-dimethylharnstoff; Phenylharnstoffe wie beispielsweise 3-(3-Chlor-p-tolyl)-1,1-dimethylharnstoff ("Chlortoluron"), 1,1-Dimethyl-3-(ααα-trifluor-m-tolyl)-harnstoff ("Fluometuron"), 3-(4-Brom-3-chlorphenyl)-1-methoxy-1-methylharnstoff ("Chlorbromuron"), 3-(4-Bromphenyl)-1-methoxy-1-methylharnstoff ("Metobromuron"), 3-(3,4-Dichlorphenyl)-1-methoxy-1-methylharnstoff ("Linuron"), 3-(4-Chlorphenyl)-1-methoxy-1-methylharnstoff ("Monolinuron"), 3-(3,4-Dichlorphenyl)-1,1-dimethylharnstoff ("Diuron"), 3-(4-Chlorphenyl-)-1,1-dimethylharnstoff ("Monuron"), 3-(3-Chlor-4-methoxyphenyl)-1,1-dimethylharnstoff ("Metoxuron"); Sulfonylharnstoffe wie beispielsweise N-(2-Chlorphenylsulfonyl)-N'-(4-methoxy-6-methyl-1,3,5-triazin-2-yl)-harnstoff, N-(2-Methoxycarbonylphenylsulfonyl)-N'-(4,6-dimethylpyrimidin-2-yl)-harnstoff, N-(2,5-Dichlorphenylsulfonyl)-N'-(4,6-dimethoxypyrimidin-2-yl)-harnstoff, N-[2-(2-butenylloxy)-phenyl-sulfonyl]-N'-(4-methoxy-6-methyl-1,3,5-triazin-2-yl)-harnstoff sowie die in den europäischen Patentpublikationen 44 808 und 44 809 genannten Sulfonylharnstoffe;

**Chloracetamide:** N-[1-Isopropyl-2-methylpropen-1-yl-(1)]-N-(2'-methoxyäthyl)-chloracetamid.

**Diphenyläther und Nitrodiphenyläther:** 2,4-Dichlorphenyl-4'-nitrophenyläther ("Nitrofen"), 2-Chlor-1-(3-äthoxy-4-nitrophenoxy)-4-trifluormethyl-benzol ("Oxyfluorfen"), 2',4'-Dichlorphenyl-3-methoxy-4-nitrophenyl-äther ("Chlormethoxynil"), 2-[4-(2,4-Dichlorphenoxy)-phenoxy)-propionsäure-methylester, N-(2-Phenoxyäthyl)-2-[5-(2-chlor-4''-trifluormethylphenoxy)-phenoxy]-propionsäureamid, 2-[2-Nitro-5-(2-chlor-4-trifluormethylphenoxy)-phenoxy]-propionsäure-2-methoxyäthylester; 2-Chlor-4-trifluormethylphenyl-3'-oxazolin-2'-yl-4'-nitrophenyläther;

**Benzoesäurederivate:** Methyl-5-(2,4-dichlorphenoxy)-2-nitrobenzoat ("Bifenox"), 5-(2-Chlor-4-trifluormethylphenoxy)-2-nitrobenzoesäure ("Acifluorfen"), 2,6-Dichlorbenzonitril ("Dichlobenil").

**Nitroaniline:** 2,6-Dinitro-N,N-dipropyl-4-trifluormethylanilin ("Trifluralin"), N-(1'-Äthylpropyl)-2,6-dinitro-3,4-xylidin ("Pendimethalin").

**Oxadiazolone:** 5-tert.-Butyl-3-(2,4-dichlor-5-isopropoxyphenyl)-1,3,4-oxadiazol-2-on ("Oxadiazon").

**Phosphate:** S-2-Methylpiperidino-carbonylmethyl-0,0-dipropyl-phosphorodithioat ("Piperophos").

**Pyrazole:** 1,3-Dimethyl-4-(2,4-dichlorbenzoyl)-5-(4-tolylsulfonyloxy)-pyrazol.

Ferner **α-(Phenoxy-phenoxy)-propionsäurederivate sowie α-(Pyridyl-2-oxy-phenoxy)-propionsäurederivate.**

Die angewendete Menge der Gegenmittel, sofern sie nicht auf die Samen gebeizt wird, schwankt zwischen etwa 0,01 und etwa 5 Gewichtsteilen Pro Gewichtsteil Herbizid. Man ermittelt von Fall zu Fall d.h. je nach verwendetem Herbizid-Typ, welches Verhältnis in Bezug auf optimale Wirkung bei der speziellen Kulturpflanze das geeignetste ist.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Für die Verwendung von Verbindungen der Formel I oder sie enthaltender Mittel zum Schützen von Kulturpflanzen gegen schädigende Wirkungen von Agrarchemikalien kommen verschiedene Methoden und Techniken in Betracht, wie beispielsweise die folgenden:

i) **Samenbeizung**

a) Beizung der Samen mit einem als Spritzpulver formulierten Wirkstoff durch Schütteln in einem Gefäss bis zur gleichmässigen Verteilung auf der Samenüberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50 - 3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die

bevorzugten Methoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) **Applikation aus Tankmischung**

Eine flüssige Aufarbeitung eines Gemisches von Gegenmittel und Herbizid (gegenseitiges Mengenverhältnis zwischen 10 : 1 und 1 : 30) wird verwendet, wobei die Aufwandmenge an Herbizid 0,1 bis 10 kg pro Hektar beträgt. Solche Tankmischung wird vorzugsweise vor oder unmittelbar nach der Aussaat appliziert oder 5 bis 10 cm tief in den noch nicht gesäten Boden eingearbeitet.

iii) **Applikation in die Saatfurche**

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) **Kontrollierte Wirkstoffabgabe**

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln Granulaten auch Verkapselungen in z. B. polymeren Stoffen in bekannter Weise verarbeitet. Die anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonid oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächeniktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind z. B. die Alkali-, Erdalkali- oder gegebenenfalls substituierten Ammoniumsalze von höheren Fettsäuren ($C_{10}$-$C_{12}$), wie z. B. die Na- oder K-Salze der Öl- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate. Die fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxiyd-Addukten. Die sulfonierten Benzimidazolderivate enthalten vorzugsweise 2 Sulfonsäuregruppen und einen Fettsäurerest mit 8 - 22 C-Atomen. Alkylarylsulfonate sind z. B. die Na-, Ca- oder

Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxyd-Adduktes oder Phospholipide in Frage.

Als nichtionisches Tensid kommen in erster Linie Polyglukolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seinen Nonylphenylpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Amnoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980 - 1981.

Die agrochemischen Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

**Emulgierbare Konzentrate**

| | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 %. |

**Stäube:**

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 %. |

**Suspension-Konzentrate**

| | |
|---|---|
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

**Benetzbare Pulver**

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 %. |

**Granulate**

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder In den folgenden Beispielen sind die Temperaturen in Celsiusgraden °C, die Drücke in Millibar mb angegeben.

**Herstellungsbeispiele**:

**Beispiel H1:**

4-Dichloracetyl-2,3-dihydro-3-methyl-1,4-benzoxazin. (Verbindung 1.1)

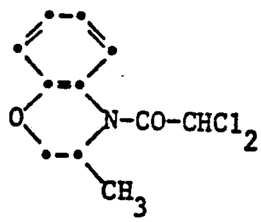

Zu einer Suspension von 7,5 g (50 mMol) 2,3-Dihydro-3-methyl-1,4-benzoxazin und 5,8 g (55 mMol) Natriumcarbonat in 120 ml Benzol lässt man unter Rühren bei einer Temperatur von 20 bis 25° C 5,3 ml (55 mMol) Dichloracetylchlorid zutropfen. Anschliessend wird das Reaktionsgemisch für 30 Minuten bei gleicher Temperatur gerührt, dann in einem Wasser/Äthylacetat-Gemisch aufgenommen. Die organische Phase wird mit gesättigter Natriumchloridlösung gewaschen, über Natriumsulfat getrocknet und eingedampft. Durch Kristallisation des Rückstands aus Diisopropyläther enthält man 10,8 g 4-Dichloracetyl-2,3-dihydro-3-methyl-1,4-benzoxazin, Smp. 105-107° C.

**Beispiel H2:**

4-(2-Chlorpropionyl)-2,3-dihydro-3-methyl-1,4-benzoxazin. (Verbindung 1.2)

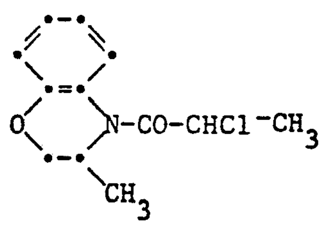

Zu einer Suspension von 7,5 g (50 mMol) 2,3-Dihydro-3-methyl-1,4-benzoxazin und 5,8 g (55 mMol) Natriumcarbonat in 120 ml Benzol lässt man unter Rühren bei einer Temperatur von 20 bis 25° C 5,35 ml (55 mMol) 2-Chlorpropionylchlorid zutropfen. Anschliessend wird das Reaktionsgemisch für 30 Minuten bei gleicher Temperatur gerührt, dann in einem Wasser/Äthylacetat-Gemisch aufgenommen. Die organische Phase wird mit gesättigter Natriumchloridlösung gewaschen, über Natriumsulfat getrocknet und eingedampft. Durch Kristallisation des Rückstands aus Diisopropyläther erhält man 19,8 g 4-(2-Chlorpropionyl)-2,3-dihydro-3-methyl-1,4-benzoxazin, Smp. 88-90° C.

In analoger Weise erhält man die in der folgenden Tabelle aufgelisteten Verbindungen.

**Tabelle 1:**

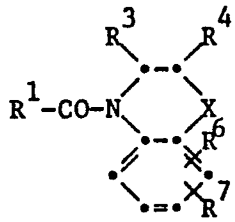

| Verb. Nr. | $R^1$ | X | $R^3$ | $R^4$ | $R^6$ | $R^7$ | Smp. [°C] |
|---|---|---|---|---|---|---|---|
| 1.1 | $CHCl_2$ | O | $CH_3$ | H | H | H | 105-107 |
| 1.2 | $CH_3$-CHCl- | O | $CH_3$ | H | H | H | 88-90 |
| 1.3 | $CH_2Cl$ | O | $CH_3$ | H | 5-$CH_3$ | H | 78-80 |
| 1.4 | $CH_2Cl$ | O | $CH_3$ | H | H | H | 88-90 |
| 1.5 | $CHCl_2$ | O | H | H | H | H | 94-96 |
| 1.6 | $CH_3$-CHCl- | O | H | H | H | H | <30 |
| 1.7 | $Cl_2C{=}CCl$- | O | H | H | H | H | <30 |
| 1.8 | $CHCl_2$ | O | H | H | 5-$CH_3$ | H | |
| 1.9 | $CH_3$-CHCl- | O | H | H | 5-$CH_3$ | H | |
| 1.10 | $Cl_2C{=}CCl$- | O | H | H | 5-$CH_3$ | H | |
| 1.11 | NC-$CH_2$- | O | H | H | 5-$CH_3$ | H | |
| 1.12 | NC-$CH_2$- | O | $CH_3$ | H | H | H | |
| 1.13 | $Cl_2C{=}CCl$- | O | $CH_3$ | H | H | H | 84-86 |
| 1.14 | $CHCl_2$ | O | $CH_3$ | H | 5-$CH_3$ | H | Öl |
| 1.15 | $CH_3$-CHCl- | O | $CH_3$ | H | 5-$CH_3$ | H | Öl |
| 1.16 | $Cl_2C{=}CCl$- | O | $CH_3$ | H | 5-$CH_3$ | H | |
| 1.17 | $CHCl_2$ | O | $CH_3$ | H | 6-$CH_3$ | H | 75-77 |
| 1.18 | $CH_3$-CHCl- | O | $CH_3$ | H | 6-$CH_3$ | H | 49-51 |
| 1.19 | $Cl_2C{=}CCl$- | O | $CH_3$ | H | 6-$CH_3$ | H | 127-129 |
| 1.20 | $CH_2Cl$- | O | $CH_3$ | H | 6-$CH_3$ | 8-$CH_3$ | Öl |
| 1.21 | $CHCl_2$- | O | $CH_3$ | H | 6-$CH_3$ | 8-$CH_3$ | 101-103 |
| 1.22 | $CH_3$-CHCl- | O | $CH_3$ | H | 6-$CH_3$ | 8-$CH_3$ | |
| 1.23 | $Cl_2C{=}CCl$- | O | $CH_3$ | H | 6-$CH_3$ | 8-$CH_3$ | |
| 1.24 | $CH_2Cl$- | O | H | -$CH_2$-$COOCH_3$ | H | H | |
| 1.25 | $CHCl_2$- | O | H | -$CH_2$-$COOCH_3$ | H | H | |
| 1.26 | $CH_3$-CHCl- | O | H | -$CH_2$-$COOCH_3$ | H | H | |
| 1.27 | $CH_2Cl$ | O | $COOCH_3$ | H | H | H | |
| 1.28 | $CHCl_2$ | O | $COOCH_3$ | H | H | H | |
| 1.29 | $CH_3$-CHCl- | O | $COOCH_3$ | H | H | H | |
| 1.30 | $CH_2Cl$ | O | $COOCH_3$ | H | 5-$CH_3$ | H | |
| 1.31 | $CHCl_2$ | O | $COOCH_3$ | H | 5-$CH_3$ | H | |
| 1.32 | $CH_3$-CHCl- | O | $COOCH_3$ | H | 5-$CH_3$ | H | |
| 1.33 | $Cl_2C{=}CCl$- | O | $COOCH_3$ | H | 5-$CH_3$ | H | |
| 1.34 | $CH_2Cl$ | S | $CH_3$ | H | H | H | |
| 1.35 | $CHCl_2$ | S | $CH_3$ | H | H | H | |
| 1.36 | $CH_3$-CHCl- | S | $CH_3$ | H | H | H | |
| 1.37 | $Cl_2C{=}CCl$- | S | $CH_3$ | H | H | H | |
| 1.38 | $ClH_2C$-$CCl_2$- | O | $CH_3$ | H | H | H | 117-119 |
| 1.39 | $CH_2Cl$- | O | H | H | 6-$CH_3$ | 8-$CH_3$ | 79-81 |
| 1.40 | $CHCl_2$- | O | H | H | 6-$CH_3$ | 8-$CH_3$ | 86-88 |
| 1.41 | $ClH_2C$-CHCl- | O | $CH_3$ | H | H | H | 69-71 |
| 1.42 | CHClF- | O | $CH_3$ | H | H | H | 97-99 |
| 1.43 | $CH_2Cl$- | S | H | H | H | H | 59-61 |
| 1.44 | $CHCl_2$- | S | H | H | H | H | 136-138 |
| 1.45 | $CH_2Cl$ | O | $CH_3$ | H | 6-Cl | H | 129-131 |
| 1.46 | $CHCl_2$ | O | $CH_3$ | H | 6-Cl | H | 123-125 |
| 1.47 | $CH_2Cl$ | O | $CH_3$ | H | 7-$CH_3$ | H | 82-84 |
| 1.48 | $CHCl_2$ | O | $CH_3$ | H | 7-$CH_3$ | H | 118-120 |
| 1.49 | $CH_2Cl$ | O | $CH_3$ | H | 6-Cl | 8-Cl | 112-114 |
| 1.50 | $CHCl_2$ | O | $CH_3$ | H | 6-Cl | 8-Cl | 205-207 |
| 1.51 | $CH_2Cl$ | O | $CH_3$ | $CH_3$ | H | H | Sdp. 119-121/0.03 mbar |

| | | | | | | | |
|---|---|---|---|---|---|---|---|
| 1.52 | $CHCl_2$ | O | $CH_3$ | $CH_3$ | H | H | Sdp. 123-125/0.03 mbar |
| 1.53 | $CHCl_2$ | S | $CH_3$ | H | H | H | 82-84 |
| 1.54 | $CHCl_2$ | O | H | $OC_2H_5$ | H | H | 76-78 |

**Formulierungsbeispiele für Wirkstoffe der Formel I oder Mischungen dieser Wirkstoffe mit Herbiziden**

| **Beispiel F1: Spritzpulver** | a) | b) | c) |
|---|---|---|---|
| Wirkstoff der Formel I oder Mischung mit Herbizid | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7 - 8 Mol ÄO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| **Beispiel F2: Emulsion-Konzentrat** | a) | b) |
|---|---|---|
| Wirkstoff der Formel I oder Mischung mit Herbizid | 10 % | 1 % |
| Octylphenolpolyäthylenglykoläther (4 - 5 Mol ÄO) | 3 % | 3 % |
| Ca-Dodecylbenzolsulfonat | 3 % | 3 % |
| Ricinusölpolyglykoläther (36 Mol ÄO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| **Beispiel F3: Stäubemittel** | a) | b) |
|---|---|---|
| Wirkstoff der Formel I oder Mischung mit Herbizid | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| **Beispiel F4: Extruder Granulat** | a) | b) |
|---|---|---|
| Wirkstoff der Formel I oder Mischung mit Herbizid | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| **Beispiel F5: Umhüllungs-Granulat** | |
|---|---|
| Wirkstoff der Formel I oder Mischung mit Herbizid | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

**Beispiel F6: Suspensions-Konzentrat**

| | a) | b) |
|---|---|---|
| Wirkstoff der Formel I oder Mischung mit Herbizid | 40 % | 5 % |
| Äthylenglykol | 10 % | 10 % |
| Nonylphenolpolyäthylenglykoläther (15 Mol ÄO) | 6 % | 1 % |
| Na-Ligninsulfonat | 10 % | 5 % |
| Carboxymethylcellulose | 1 % | 1 % |
| 37 %-ige wässrige Formaldehyd-Lösung | 0,2 % | 0,2 % |
| Silikonöl in Form einer 75 %-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel F7: Salzlösung**

| | |
|---|---|
| Wirkstoff der Formel I oder Mischung mit Herbizid | 5 % |
| Isopropylamin | 1 % |
| Octylphenolpolyäthylenglykoläther (78 Mol ÄO) | 3 % |
| Wasser | 91 %. |

**Biologische Beispiele**

Die Fähigkeit der Verbindungan der Formel I, Kulturpflanzen vor der phytotoxischen Wirkung starker Herbizide zu schützen, kann aus den folgenden Beispielen ersehen werden. In der Versuchsbeschreibung werden die Verbindungen der Formel I als Safener oder Gegenmittel (Antidote) bezeichnet.

**Beispiel B1:**

Versuch mit Herbizid und Gegenmittel in Mais. Herbizid und Gegenmittel werden zusammen als Tankmischung im Vorauflaufverfahren appliziert.

Plastikcontainer (25 cm lang x 17 cm breit x 12 cm hoch) werden mit sandiger Lehmerde gefüllt und Maissamen der Sorte LG 5 eingesät. Nach dem Bedecken der Samen wird die als Safener zu prüfende Substanz zusammen mit dem Herbizid in verdünnter Lösung als Tankmischung auf die Bodenoberfläche gesprüht. 21 Tage nach der Applikation wird die Schutzwirkung des Safeners in Prozent ausgewertet. Als Referenz dienen dabei die mit dem Herbizid allein behandelten Pflanzen (keine Schutzwirkung) sowie die vollständig unbehandelte Kontrolle. (100 %-ige Schutzwirkung)

**Testresultate:**

**Herbizid:** H-Chloracetyl-N-(2-methoxy-1-methyläthyl)-2,6-dimethylanilin.

| Gegenmittel | | Herbizid | relative Schutzwirkung |
|---|---|---|---|
| No. | kg/ha | kg/ha | in % |
| 1.1 | 1,5 | 6 | 63 |
| 1.1 | 0,75 | 6 | 63 |
| 1.1 | 1 | 4 | 50 |
| 1.1 | 0,5 | 4 | 63 |
| 1.2 | 1,5 | 6 | 63 |
| 1.2 | 0,75 | 6 | 63 |
| 1.2 | 1 | 4 | 63 |
| 1.2 | 0,5 | 4 | 63 |

**Beispiel B2:**

Vergleichsversuche zwischen einem erfindungsgemässen Gegenmittel und einem bekannten Gegenmittel.

In der in Beispiel B1 beschriebenen Versuchsanordnung wird im Vergleich die Schutzwirkung des erfindungsgemässen Wirkstoffs

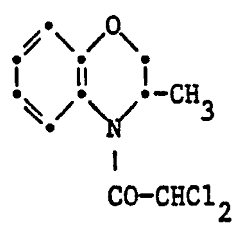

Nr. 1.1

und des in der DE-OS-2 828 293 beschriebenen Gegenmittels

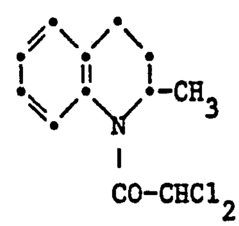

Verb. A

bestimmt. Als Herbizide werden dabei eingesetzt: N-Chloracetyl-N-(2-methoxy-1-methyläthyl)-2,6-dimethylanilin [Herbizid AA] und N-Chloracetyl-N-(2-methoxy-1-methyläthyl)-2-äthyl-6-methylanilin [Herbizid BB]. Als Versuchspflanze wird Mais der Zuchtlinie "00482-0220 B" verwendet. Die Applikation wird im Vorauflauf als Tankmischung von Herbizid als Gegenmittel vorgenommen. Die Auswertung erfolgt wie im Versuch B1 3 Wochen nach Applikation in Prozent Schutzwirkung.

**Testresultate:**

| Gegenmittel | | Herbizid | relative Schutzwirkung in % | |
|---|---|---|---|---|
| No. | hg/ha | kg/ha | gegen Herbizid AA | gegen Herbizid BB |
| 1.1 | 1.5 | 6 | 88 | 88 |
| 1.1 | 0.38 | 6 | 75 | 88 |
| 1.1 | 1 | 4 | 88 | 63 |
| 1.1 | 0.25 | 4 | 88 | 75 |
| Verb. A | 1.5 | 6 | 50 | 75 |
| Verb. A | 0.38 | 6 | 13 | 50 |
| Verb. A | 1 | 4 | 63 | 38 |
| Verb. A | 0.25 | 4 | 50 | 13 |

Der Vergleich der Wirkung der Gegenmittel Nr. 1.1 und Verbindung A auf die Maissaat bei der Behandlung mit den Herbiziden AA und BB zeigt, dass der Wirkstoff Nr. 1.1 bei allen geprüften Mischungsverhältnissen und Aufwandmengen stets eine bessere Schutzwirkung als die Verbindung A entfaltet. Dabei wird die Schädigung der Kultur bei Anwendung des Wirkstoffs 1.1 stets auf ein tolerierbares Mass zurückdrängt, während bei Verwendung von Verbindung A die Schutzwirkung für agrartechnische Zwecke meist ungenügend bleibt.

**Patentansprüche** für die Vertragsstaaten: BE, CH, LI, DE, FR, IT, NL, GB

1. Ein Mittel zum Schützen von Kulturpflanzen vor der phytotoxischen Wirkung von Herbiziden, dadurch gekennzeichnet, dass es neben inerten Trägermaterial und gegebenenfalls dem Herbizid als antagonisierenden Wirkstoff ein Acylamid-Derivat der Formel I

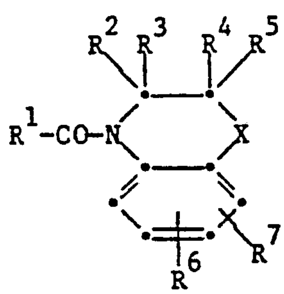

(I) ,

enthält, worin

X Sauerstoff, Schwefel, -SO- oder $-SO_2-$,

$R^1$ $C_1$-$C_6$-Halogenalkyl, $C_1$-$C_6$-Cyanalkyl oder $C_2$-$C_6$-Halogenalkenyl,

$R^2$ und $R^5$ unabhängig voneinander Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R^3$ und $R^4$ unabhängig voneinander Wasserstoff, $C_2$-$C_4$-Alkenyl, $C_2$-$C_4$-Alkinyl, Cyan, $C_2$-$C_4$-Alkoxyalkyl, $-COOR^8$, $-CO-NR^9R^{10}$ oder unsubstituiertes oder durch Halogen, Cyan oder -CO-A substituiertes $C_1$-$C_4$-Alkyl,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl oder $C_1$-$C_4$-Halogenalkoxy,

A $C_1$-$C_4$-Alkyl, $C_2$-$C_4$-Alkenyl, $C_1$-$C_4$-Alkoxy, $C_3$-$C_4$-Alkenyloxy, $C_3$-$C_4$-Alkinyloxy oder $-NR^{11}R^{12}$,

$R^8$, $R^{10}$ und $R^{12}$ unabhängig voneinander Wasserstoff, $C_1$-$C_4$-Alkyl, $C_3$-$C_4$-Alkenyl, $C_3$-$C_4$-Alkinyl oder $C_3$-$C_4$-Alkoxyalkyl und

$R^9$ und $R^{11}$ unabhängig voneinander Wasserstoff, $C_1$-$C_4$-Alkyl, $C_3$-$C_4$-Alkenyl, $C_1$-$C_4$-Alkoxy, $C_3$-$C_4$-Alkinyl oder $C_3$-$C_4$-Alkoxyalkyl bedeuten, wobei auch

$R^9$ and $R^{10}$ sowie $R^{11}$ und $R^{12}$ zusammen mit dem sie bindenden Stickstoffatom einen 5- bis 6-gliedrigen gesättigten Heterocyclus bilden können, der als Ringglied gegebenenfalls ein Sauerstoff- oder Schwefelatom oder eine -NH- oder $-N(C_1$-$C_4$-Alkyl)-Brücke enthalten kann.

2. Mittel gemäss Anspruch 1 dadurch gekennzeichnet, dass X für Sauerstoff steht.

3. Mittel gemäss Anspruch 1, dadurch gekennzeichnet, dass $R^1$ für $C_1$-$C_6$-Halogenalkyl oder $C_2$-$C_6$-Halogenalkenyl steht.

4. Mittel gemäss Anspruch 1, dadurch gekennzeichnet, dass $R^2$ und $R^5$ Wasserstoff bedeuten.

5. Mittel gemäss Anspruch 1, dadurch gekennzeichnet, dass $R^3$ und $R^4$ unabhängig voneinander Wasserstoff,

$C_1$-$C_4$-Alkyl, $C_1$-$C_4$-Alkoxycarbonylmethyl oder $C_1$-$C_4$-Alkoxycarbonyl bedeuten.

6. Mittel gemäss Anspruch 1, dadurch gekennzeichnet, dass $R^6$ und $R^7$ unabhängig voneinander Wasserstoff oder $C_1$-$C_4$-Alkyl bedeuten.

7. Mittel gemäss Anspruch 5, dadurch gekennzeichnet, dass $R^3$ und $R^4$ unabhängig voneinander Wasserstoff oder Methyl bedeuten.

8. Mittel gemäss Anspruch 3, dadurch gekennzeichnet, dass $R^1$ für $C_1$-$C_4$-Halogenalkyl steht.

9. Mittel gemäss Anspruch 3, dadurch gekennzeichnet, dass $R^1$ für Dichlormethyl steht.

10. Mittel gemäss Anspruch 1, dadurch gekennzeichnet, dass X Sauerstoff, $R^1$ $C_1$-$C_6$-Halogenalkyl oder $C_2$-$C_6$-Halogenalkenyl, $R^2$ und $R^5$ Wasserstoff, $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl und $R^6$ und $R^7$ unabhängig voneinander Wasserstoff oder $C_1$-$C_4$-Alkyl bedeuten.

11. Mittel gemäss Anspruch 9, dadurch gekennzeichnet, dass $R^1$ für $C_1$-$C_4$-Halogenalkyl und $R^3$, $R^4$, $R^6$ und $R^7$ unabhängig voneinander für Wasserstoff oder Methyl stehen.

12. Mittel gemäss Anspruch 11, dadurch gekennzeichnet, dass $R^1$ für Dichlormethyl steht.

13. Mittel gemäss Anspruch 1, dadurch gekennzeichnet, dass es als antagonisierenden Wirkstoff 4-Dichloracetyl-2,3-dihydro-3-methyl-1,4-benzoxazin, 4-Dichloracetyl-2,3-dihydro-3,6-dimethyl-1,4-benzoxazin oder 4-(2-Chlorpropionyl)-2,3-dihydro-3-methyl-1,4-benzoxazin enthält.

14. Neue Acylamid-Derivate der Formel I

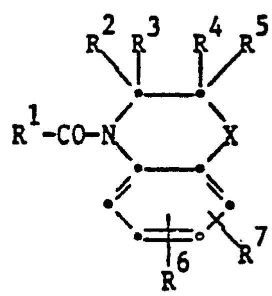

(I) ,

worin

X Sauerstoff, Schwefel, -SO- oder -$SO_2$-,

$R^1$ $C_1$-$C_6$-Cyanalkyl, $C_2$-$C_6$-Halogenalkenyl oder durch mindestens zwei Halogenatome substituiertes $C_1$-$C_6$-Alkyl,

$R^2$ und $R^5$ unahängig voneinander Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R^3$ und $R^4$ unabhängig voneinander Wasserstoff, $C_2$-$C_4$-Alkenyl, $C_2$-$C_4$-Alkinyl, Cyan, $C_2$-$C_4$-Alkoxyalkyl, -$COOR^8$, -CO-$NR^9R^{10}$ oder unsubstituiertes oder durch Halogen, Cyan oder -CO-A substituierter $C_1$-$C_4$-Alkyl,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl oder $C_1$-$C_4$-Halogenalkoxy,

A $C_1$-$C_4$-Alkyl, $C_2$-$C_4$-Alkenyl, $C_1$-$C_4$-Alkoxy, $C_3$-$C_4$-Alkenyloxy, $C_3$-$C_4$-Alkinyloxy oder -$NR^{11}R^{12}$,

$R^8$, $R^{10}$ und $R^{12}$ unabhängig voneinander Wasserstoff, $C_1$-$C_4$-Alkyl, $C_3$-$C_4$-Alkenyl $C_3$-$C_4$-Alkinyl oder $C_3$-$C_4$-Alkoxyalkyl und

$R^9$ und $R^{11}$ unabhängig voneinander Wasserstoff, $C_1$-$C_4$-Alkyl, $C_3$-$C_4$-Alkenyl, $C_1$-$C_4$-Alkoxy, $C_3$-$C_4$-Alkinyl oder $C_3$-$C_4$-Alkoxyalkyl bedeuten, wobei auch

$R^9$ and $R^{10}$ sowie $R^{11}$ und $R^{12}$ zusammen mit dem sie bindenden Stickstoffatom einen 5- bis 6-gliedrigen gesättigten Heterocyclus bilden können, der als Ringglied gegebenenfalls ein Sauerstoff- oder Schwefelatom oder eine -NH- oder -N($C_1$-$C_4$-Alkyl)-Brücke enthalten kann; unter Ausnahme der Verbindung 4-Trifluoracetyl-2,3-dihydro-1,4-benzothiazin.

15. Verbindungen gemäss Anspruch 14, dadurch gekennzeichnet, dass X für Sauerstoff steht.

16. Verbindungen gemäss Anspruch 14, dadurch gekennzeichnet, dass $R^1$ für $C_2$-$C_6$-Halogenalkenyl oder durch mindestens zwei Halogenatome substituiertes $C_1$-$C_6$-Alkyl steht.

17. Verbindungen gemäss Anspruch 14, dadurch gekennzeichnet, dass $R^2$ und $R^5$ Wasserstoff bedeuten.

18. Verbindungen gemäss Anspruch 14, dadurch gekennzeichnet, dass $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl bedeuten.

19. Verbindungen gemäss Anspruch 14, dadurch gekennzeichnet, dass $R^6$ und $R^7$ unabhängig voneinander Wasserstoff oder $C_1$-$C_4$-Alkyl bedeuten.

20. Verbindungen gemäss Anspruch 18, dadurch gekennzeichnet, dass $R^3$ und $R^4$ unabhängig voneinander Wasserstoff oder Methyl bedeuten.

21. Verbindungen gemäss Anspruch 16, dadurch gekennzeichnet, dass $R^1$ für durch mindestens zwei Halogenatome substituiertes $C_1$-$C_4$-Alkyl steht.

22. Verbindungen gemäss Anspruch 21, dadurch gekennzeichnet, dass $R^1$ für Dichlormethyl steht.

23. Verbindungen gemäss Anspruch 14, dadurch gekennzeichnet, dass X Sauerstoff, $R^1$ $C_2$-$C_6$-Halogenalkyl, oder durch mindestens zwei Halogenatome substituiertes $C^1$-$C_4$-Alkyl, $R^2$ und $R^5$ Wasserstoff, $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl und

$R^6$ und $R^7$ unabhängig voneinander Wasserstoff oder $C_1$-$C_4$-Alkyl bedeuten.

24. Verbindungen gemäss Anspruch 23, dadurch gekennzeichnet, dass $R^1$ für durch mindestens zwei Halogenatome substituiertes $C_1$-$C_4$-Alkyl und $R^3$, $R^4$, $R^6$ und $R^7$ unabhängig voneinander für Wasserstoff oder Methyl stehen.

25. Verbindungen gemäss Anspruch 24, dadurch gekennzeichnet, dass $R^1$ für Dichlormethyl steht.

26. 4-Dichloracetyl-2,3-dihydro-3-methyl-1,4-benzoxazin gemäss Anspruch 14.

27. 4-Dichloracetyl-2,3-dihydro-3,6-dimethyl-1,4-benzoxazin gemäss Anspruch 14.

28. Verfahren zur selektiven Bekämpfung von Unkräutern in Nutzpflanzenkulturen, dadurch gekennzeichnet, dass man die Kulturpflanzen oder deren Anbaufläche sowohl mit einem Herbizid, als auch einer wirksamen Menge eines Acylamid-Derivates der Formel I gemäss Anspruch 1 als Gegenmittel behandelt.

29. Verwendung der Acylamid-Derivate der Formel 1 gemäss Anspruch 1 zum Schützen von Kulturpflanzen gegen die schädigende Wirkung von Herbiziden.

30. Verfahren zum Schützen von Kulturpflanzen vor Schäden, die bei der Applikation von Herbiziden auftreten, dadurch gekennzeichnet, dass man entweder die Anbaufläche für die Pflanze vor oder während der Applikation des Herbizids oder den Samen oder die Stecklinge der Pflanzen oder die Pflanze selbst mit einer wirksamen Menge eines Acylamid-Derivat der Formel 1 gemäss Anspruch 1 behandelt.

31. Saatgut von Nutzpflanzen, das mit einer antagonistisch wirksamen Menge eines Acylamid-Derivates der Formel I gemäss Anspruch 1 behandelt worden ist.

32. Verfahren zur Herstellung der Verbindungen der Formel I, worin $R^1$ $C_1$-$C_6$-Cyanalkyl, $C_2$-$C_6$-Halogenalkenyl oder durch mindestens zwei Halogenatome substituiertes $C_1$-$C_6$-Halogenalkyl bedeutet, gemäss Anspruch 14, dadurch gekennzeichnet, dass man ein Acylhalogenid der Formel II

$R^1$ - CO - Hal (II).

worin $R^1$ $C_1$-$C_6$-Cyanalkyl, $C_2$-$C_6$-Halogenalkenyl oder durch mindestens zwei Halogenatome substituiertes $C_1$-$C_6$-Alkyl und Hal Chlor oder Brom bedeuten, in Gegenwart eines säurebindenden Mittels mit einem Amin der Formel III

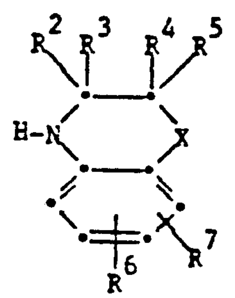

(III),

worin $R^2$, $R^3$, $R^4$, $R^5$, $R^6$, $R^7$ und X die unter Formel I gegebene Bedeutung haben, umsetzt, mit der Massgabe dass $R^1$ nicht für Trifluormethyl stehen darf, wenn glechzeitig $R^2$, $R^3$, $R^4$, $R^5$, $R^6$ und $R^7$ Wasserstoff und X Schwefel bedeuten.

**Patentansprüche** für den Vertragsstaat: AT

1. Ein Mittel zum Schützen von Kulturpflanzen vor der phytotoxischen Wirkung von Herbiziden, dadurch gekennzeichnet, dass es neben inerten Trägermaterial und gegebenenfalls dem Herbizid als antagonisierenden Wirkstoff ein Acylamid-Derivat der Formel I

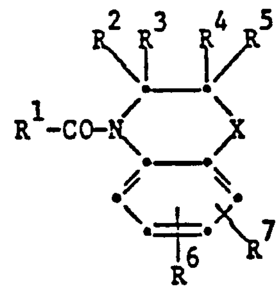

(I) ,

enthält, worin

X Sauerstoff, Schwefel, -SO- oder $-SO_2-$,

$R^1$ $C_1$-$C_6$-Halogenalkyl, $C_1$-$C_6$-Cyanalkyl oder $C_2$-$C_6$-Halogenalkenyl,

$R^2$ und $R^5$ unabhängig voneinander Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R^3$ und $R^4$ unabhängig voneinander Wasserstoff, $C_2$-$C_4$-Alkenyl, $C_2$-$C_4$-Alkinyl, Cyan, $C_2$-$C_4$-Alkoxyalkyl, $-COOR^8$, $-CO-NR^9R^{10}$ oder unsubstituiertes oder durch Halogen, Cyan oder -CO-A substituiertes $C_1$-$C_4$-Alkyl,

$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl oder $C_1$-$C_4$-Halogenalkoxy,

A $C_1$-$C_4$-Alkyl, $C_2$-$C_4$-Alkenyl, $C_1$-$C_4$-Alkoxy, $C_3$-$C_4$-Alkenyloxy, $C_3$-$C_4$-Alkinyloxy oder $-NR^{11}R^{12}$,

$R^8$, $R^{10}$ und $R^{12}$ unabhängig voneinander Wasserstoff, $C_1$-$C_4$-Alkyl, $C_3$-$C_4$-Alkenyl, $C_3$-$C_4$-Alkinyl oder $C_3$-$C_4$-Alkoxyalkyl und

$R^9$ und $R^{11}$ unabhängig voneinander Wasserstoff, $C_1$-$C_4$-Alkyl, $C_3$-$C_4$-Alkenyl, $C_1$-$C_4$-Alkoxy, $C_3$-$C_4$-Alkinyl oder $C_3$-$C_4$-Alkoxyalkyl bedeuten, wobei auch

$R^9$ and $R^{10}$ sowie $R^{11}$ und $R^{12}$ zusammen mit dem sie bindenden Stickstoffatom einen 5- bis 6-gliedrigen gesättigten Heterocyclus bilden können, der als Ringglied gegebenenfalls ein Sauerstoff- oder Schwefelatom oder eine -NH- oder $-N(C_1$-$C_4$-Alkyl)-Brücke enthalten kann.

2. Mittel gemäss Anspruch 1 dadurch gekennzeichnet, dass X für Sauerstoff steht.

3. Mittel gemäss Anspruch 1, dadurch gekennzeichnet, dass $R^1$ für $C_1$-$C_6$-Halogenalkyl oder $C_2$-$C_6$-Halogenalkenyl steht.

4. Mittel gemäss Anspruch 1, dadurch gekennzeichnet, dass $R^2$ und $R^5$ Wasserstoff bedeuten.

5. Mittel gemäss Anspruch 1, dadurch gekennzeichnet, dass $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl bedeuten.

6. Mittel gemäss Anspruch 1, dadurch gekennzeichnet, dass $R^6$ und $R^7$ unabhängig voneinander Wasserstoff oder $C_1$-$C_4$-Alkyl bedeuten.

7. Mittel gemäss Anspruch 5, dadurch gekennzeichnet, dass $R^3$ und $R^4$ unabhängig voneinander Wasserstoff oder Methyl bedeuten.

8. Mittel gemäss Anspruch 3, dadurch gekennzeichnet, dass $R^1$ für $C_1$-$C_4$-Halogenalkyl steht.

9. Mittel gemäss Anspruch 3, dadurch gekennzeichnet, dass $R^1$ für Dichlormethyl steht.

10. Mittel gemäss Anspruch 1, dadurch gekennzeichnet, dass X Sauerstoff, $R^1$ $C_1$-$C_6$-Halogenalkyl oder $C_2$-$C_6$-Halogenalkenyl, $R^2$ und $R^5$ Wasserstoff, $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl und $R^6$ und $R^7$ unabhängig voneinander Wasserstoff oder $C_1$-$C_4$-Alkyl bedeuten.

11. Mittel gemäss Anspruch 9, dadurch gekennzeichnet, dass $R^1$ für $C_1$-$C_4$-Halogenalkyl und $R^3$, $R^4$, R6 und $R^7$ unabhängig voneinander für Wasserstoff oder Methyl stehen.

12. Mittel gemäss Anspruch 11, dadurch gekennzeichnet, dass $R^1$ für Dichlormethyl steht.

13. Mittel gemäss Anspruch 1, dadurch gekennzeichnet, dass es als antagonisierenden Wirkstoff 4-Dichloracetyl-2,3-dihydro-3-methyl-1,4-benzoxazin, 4-Dichloracetyl-2,3-dihydro-3,6-dimethyl-1,4-benzoxazin oder 4-(2-Chlorpropionyl)-2,3-dihydro-3-methyl-1,4-benzoxazin enthält.

14. Verfahren zur Herstellung der Acylamid-Derivate der Formel I

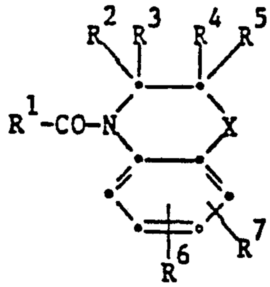

(I) ,

worin
X Sauerstoff, Schwefel, -SO- oder $-SO_2-$,
$R^1$ $C_1$-$C_6$-Cyanalkyl, $C_2$-$C_6$-Halogenalkenyl oder durch mindestens zwei Halogenatome substituiertes $C_1$-$C_6$-Alkyl,
$R^2$ und $R^5$ unabhängig voneinander Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,
$R^3$ und $R^4$ unabhängig voneinander Wasserstoff, $C_2$-$C_4$-Alkenyl, $C_2$-$C_4$-Alkinyl, Cyan, $C_2$-$C_4$-Alkoxyalkyl, $-COOR^8$, $-CO-NR^9R^{10}$ oder unsubstituiertes oder durch Halogen, Cyan oder -CO-A substituierter $C_1$-$C_4$-Alkyl,
$R^6$ und $R^7$ unabhängig voneinander Wasserstoff, Halogen, $C_1$-$C_4$-Alkyl, $C_1$-$C_4$-Alkoxy, $C_1$-$C_4$-Halogenalkyl oder $C_1$-$C_4$-Halogenalkoxy,
A $C_1$-$C_4$-Alkyl, $C_2$-$C_4$-Alkenyl, $C_1$-$C_4$-Alkoxy, $C_3$-$C_4$-Alkenyloxy, $C_3$-$C_4$-Alkinylkoxy oder $-NR^{11}R^{12}$,
$R^8$, $R^{10}$ und $R^{12}$ unabhängig voneinander Wasserstoff, $C_1$-$C_4$-Alkyl, $C_3$-$C_4$-Alkenyl $C_3$-$C_4$-Alkinyl oder $C_1$-$C_4$-Alkoxyalkyl und
$R^9$ und $R^{11}$ unabhängig voneinander Wasserstoff, $C_1$-$C_4$-Alkyl, $C_3$-$C_4$-Alkenyl, $C_1$-$C_4$-Alkoxy, $C_3$-$C_4$-Alkinyl oder $C_3$-$C_4$-Alkoxyalkyl bedeuten, wobei auch
$R^9$ and $R^{10}$ sowie $R^{11}$ und $R^{12}$ zusammen mit dem sie bindenden Stickstoffatom einen 5- bis 6-gliedrigen gesättigten Heterocyclus bilden können, der als Ringglied gegebenenfalls ein Sauerstoff- oder Schwefelatom oder eine -NH- oder $-N(C_1$-$C_4$-Alkyl)-Brücke enthalten kann, unter Ausnahme der Verbindung 4-Trifluoracetyl-2,3-dihydro-1,4-benzothiazin, dadurch gekennzeichnet, dass man ein Acylhalogenid der Formel II

$R^1$ - CO - Hal (II),

worin $R^1$ $C_1$-$C_6$-Cyanalkyl, $C_2$-$C_6$-Halogenalkenyl oder durch mindestens zwei Halogenatome substituiertes $C_1$-$C_6$-Alkyl und Hal Chlor oder Brom bedeuten in Gegenwart eines Säurebindenden Mittels mit einem Amin der Formel III

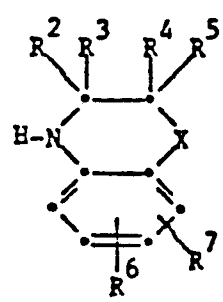

(III),

worin $R^2$, $R^3$, $R^4$, $R^5$, $R^6$, $R^7$ und X die unter Formel I gegebene Bedeutung haben, umsetzt, mit der Massgabe, dass $R^1$ nicht für Trifluormethyl stehen darf, wenn gleichzeitig $R^2$, $R^3$, $R^4$, $R^5$, $R^6$ und $R^7$ Wasserstoff und X Schwefel bedeuten.

15. Verfahren gemäss Anspruch 14, dadurch gekennzeichnet, dass X für Sauerstoff steht.

16. Verfahren gemäss Anspruch 14, dadurch gekennzeichnet, dass $R^1$ für $C_2$-$C_6$-Halogenalkenyl oder durch mindestens zwei Halogenatome substituiertes $C_1$-$C_6$-Alkyl steht.

17. Verfahren gemäss Anspruch 14, dadurch gekennzeichnet, dass $R^2$ und $R^5$ Wasserstoff bedeuten.

18. Verfahren gemäss Anspruch 14, dadurch gekennzeichnet, dass $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl bedeuten.

19. Verfahren gemäss Anspruch 14, dadurch gekennzeichnet, dass $R^6$ und $R^7$ unabhängig voneinander Wasserstoff oder $C_1$-$C_4$-Alkyl bedeuten.

20. Verfahren gemäss Anspruch 18, dadurch gekennzeichnet, dass $R^3$ und $R^4$ unabhängig voneinander

Wasserstoff oder Methyl bedeuten.

21. Verfahren gemäss Anspruch 16, dadurch gekennzeichnet, dass $R^1$ für durch mindestens zwei Halogenatome substituiertes $C_1$-$C_4$-Alkyl steht.

22. Verfahren gemäss Anspruch 21, dadurch gekennzeichnet, dass $R^1$ für Dichlormethyl steht.

23. Verfahren gemäss Anspruch 14, dadurch gekennzeichnet, dass X Sauerstoff, $R^1$ $C_2$-$C_6$-Halogenalkyl, oder durch mindestens zwei Halogenatome substituiertes $C_1$-$C_4$-Alkyl, $R^2$ und $R^5$ Wasserstoff, $R^3$ und $R^4$ unabhängig voneinander Wasserstoff, $C_1$-$C_4$-Alkyl, $C_1$-$C_4$-Alkoxycarbonylmethyl oder $C_1$-$C_4$-Alkoxycarbonyl und $R^6$ und $R^7$ unabhängig voneinander Wasserstoff oder $C_1$-$C_4$-Alkyl bedeuten.

24. Verbindungen gemäss Anspruch 23, dadurch gekennzeichnet, dass $R^1$ für durch mindestens zwei Halogenatome substituiertes $C_1$-$C_4$-Alkyl und $R^3$ $R^4$, $R^6$ und $R^7$ unabhängig voneinander für Wasserstoff oder Methyl stehen.

25. Verfahren gemäss Anspruch 24, dadurch gekennzeichnet, dass $R^1$ für Dichlormethyl steht.

26. Verfahren gemäss Anspruch 14, dadurch gekennzeichnet, dass man 4-Dichloracetyl-2,3-dihydro-3-methyl-1,4-benzoxazin herstellt.

27. Verfahren gemäss Anspruch 14, dadurch gekennzeichnet, dass man 4-Dichloracetyl-2,3-dihydro-3,6-dimethyl-1,4-benzoxazin herstellt.

28. Verfahren zur selektiven Bekämpfung von Unkräutern in Nutz-Pflanzenkulturen, dadurch gekennzeichnet, dass man die Kulturpflanzen oder deren Anbaufläche sowohl mit einem Herbizid, als auch einer wirksamen Menge eines Acylamid-Derivates der Formel I gemäss Anspruch 1 als Gegenmittel behandelt.

29. Verwendung der Acylamid-Derivate der Formel 1 gemäss Anspruch 1 zum Schützen von Kulturpflanzen gegen die schädigende Wirkung von Herbiziden.

30. Verfahren zum Schützen von Kulturpflanzen vor Schäden, die bei der Applikation von Herbiziden auftreten, dadurch gekennzeichnet, dass man entweder die Anbaufläche für die Pflanze vor oder während der Applikation des Herbizids oder den Samen oder die Stecklinge der Pflanzen oder die Pflanze selbst mit einer wirksamen Menge eines Acylamid-Derivat der Formel I gemäss Anspruch 1 behandelt.

**Claims** for the contracting states: BE, CH, LI, DE, FR, IT, NL, GB

1. A composition for protecting cultivated plants against the phytotoxic action of herbicides, characterised in that, in addition to inert carrier material and, if desired, the herbicide, it contains, as the antagonising active substance, an acylamide derivative of the formula I

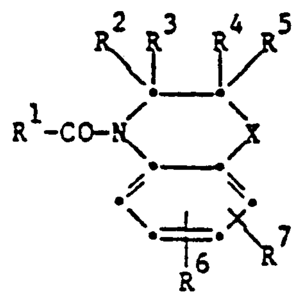

(I)

in which

X is oxygen, sulphur, -SO- or $-SO_2-$,

$R^1$ is $C_1$-$C_6$-haloalkyl, $C_1$-$C_6$-cyanoalkyl or $C_2$-$C_6$-haloalkenyl,

$R^2$ and $R^5$ independently of one another are hydrogen, $C_1$-$C_4$-alkyl, $C_1$-$C_4$-haloalkyl, $C_2$-$C_4$-alkenyl, $C_2$-$C_4$-alkynyl or $C_2$-$C_4$-alkoxyalkyl,

$R^3$ and $R^4$ independently of one another are hydrogen, $C_2$-$C_4$-alkenyl, $C_2$-$C_4$-alkynyl, cyano, $C_2$-$C_4$-alkoxyalkyl, $-COOR^8$, $-CO-NR^9R^{10}$, or $C_1$-$C_4$-alkyl which is unsubstituted or substituted by halogen, cyano or by -CO-A,

$R^6$ and $R^7$ independently of one another are hydrogen, halogen, $C_1$-$C_4$-alkyl, $C_1$-$C_4$-alkoxy, $C_1$-$C_4$-haloalkyl or $C_1$-$C_4$-haloalkoxy,

A is $C_1$-$C_4$-alkyl, $C_2$-$C_4$-alkenyl, $C_1$-$C_4$-alkoxy, $C_3$-$C_4$-alkenyloxy, $C_3$-$C_4$-alkynyloxy or $-NR^{11}R^{12}$,

$R^8$, $R^{10}$ and $R^{12}$ independently of one another are hydrogen, $C_1$-$C_4$-alkyl, $C_3$-$C_4$-alkenyl, $C_3$-$C_4$-alkynyl or $C_3$-$C_4$-alkoxyalkyl, and

$R^9$ and $R^{11}$ independently of one another are hydrogen, $C_1$-$C_4$-alkyl, $C_3$-$C_4$-alkenyl, $C_1$-$C_4$-alkoxy, $C_3$-$C_4$-alkynyl or $C_3$-$C_4$-alkoxyalkyl,

it being also possible for $R^9$ and $R^{10}$ and also $R^{11}$ and $R^{12}$, together with the nitrogen atom linking them, to form a 5-membered or 6-membered saturated heterocyclic structure which can, if desired, contain an oxygen or sulphur atom or an -NH- or $-N(C_1$-$C_4$-alkyl)- bridge as a member of the ring.

2. A composition according to claim 1, characterised in that X is oxygen.

3. A composition according to claim 1, characterised in that $R^1$ is $C_1$-$C_6$-haloalkyl or $C_2$-$C_6$-haloalkenyl.

4. A composition according to claim 1, characterised in that $R^2$ and $R^5$ are hydrogen.

5. A composition according to claim 1, characterised in that $R^3$ and $R^4$ independently of one another are hydrogen, $C_1$-$C_4$-alkyl, $C_1$-$C_4$-alkoxycarbonylmethyl or $C_1$-$C_4$-alkoxy-carbonyl.

6. A composition according to claim 1, characterised in that $R^6$ and $R^7$ independently of one another are hydrogen or $C_1$-$C_4$-alkyl.

7. A composition according to claim 5, characterised in that $R^3$ and $R^4$ independently of one another are hydrogen or methyl.

8. A composition according to claim 3, characterised in that $R^1$ is $C_1$-$C_4$-haloalkyl.

9. A composition according to claim 3, characterised in that $R^1$ is dichloromethyl.

10. A composition according to claim 1, characterised in that X is oxygen, $R^1$ is $C_1$-$C_6$-haloalkyl or $C_2$-$C_6$-haloalkenyl, $R^2$ and $R^5$ are hydrogen, $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl and $R^6$ and $R^7$ independently of one another are hydrogen or $C_1$-$C_4$-alkyl.

11. A composition according to claim 9, characterised in that $R^1$ is $C_1$-$C_4$-haloalkyl and $R^3$, $R^4$, $R^6$ and $R^7$ independently of one another are hydrogen or methyl.

12. A composition according to claim 11, characterised in that $R^1$ is dichloromethyl.

13. A composition according to claim 1, characterised in that it contains as the antagonising active substance, 4-dichloroacetyl-2,3-dihydro-3-methyl-1,4-benzoxazine, 4-dichloroacetyl-2,3-dihydro-3,6-dimethyl-1,4-benzoxazine or 4-(2-chloropropionyl)-2,3-dihydro-3-methyl-1,4-benzoxazine.

14. Novel acylamide derivatives of the formula I

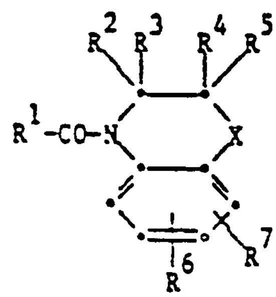

(I)

in which

X is oxygen, sulphur, -SO- or -$SO_2$-,

$R^1$ is $C_1$-$C_6$-cyanoalkyl, $C_2$-$C_6$-haloalkenyl, or $C_1$-$C_6$-alkyl which is substituted by at least two halogen atoms,

$R^2$ and $R^5$ independently of one another are hydrogen, $C_1$-$C_4$-alkyl, $C_1$-$C_4$-haloalkyl, $C_2$-$C_4$-alkenyl, $C_2$-$C_4$-alkynyl or $C_2$-$C_4$-alkoxyalkyl,

$R^3$ and $R^4$ independently of one another are hydrogen, $C_2$-$C_4$-alkenyl, $C_2$-$C_4$-alkynyl, cyano, $C_2$-$C_4$-alkoxyalkyl, -$COOR^8$, -CO-$NR^9R^{10}$, or $C_1$-$C_4$-alkyl which is unsubstituted or substituted by halogen, cyano or by -CO-A,

$R^6$ and $R^7$ independently of one another are hydrogen, halogen, $C_1$-$C_4$-alkyl, $C_1$-$C_4$-alkoxy, $C_1$-$C_4$-haloalkyl or $C_1$-$C_4$-haloalkoxy,

A is $C_1$-$C_4$-alkyl, $C_2$-$C_4$-alkenyl, $C_1$-$C_4$-alkoxy, $C_3$-$C_4$-alkenyloxy, $C_3$-$C_4$-alkynyloxy or -$NR^{11}R^{12}$,

$R^8$, $R^{10}$ and $R^{12}$ independently of one another are hydrogen, $C_1$-$C_4$-alkyl, $C_3$-$C_4$-alkenyl, $C_3$-$C_4$-alkynyl or $C_3$-$C_4$-alkoxyalkyl, and

$R^9$ and $R^{11}$ independently of one another are hydrogen, $C_1$-$C_4$-alkyl, $C_3$-$C_4$-alkenyl, $C_1$-$C_4$-alkoxy, $C_3$-$C_4$-alkynyl or $C_3$-$C_4$-alkoxyalkyl,

it being also possible for $R^9$ and $R^{10}$ and also $R^{11}$ and $R^{12}$, together with the nitrogen atom linking them, to form a 5-membered or 6-membered saturated heterocyclic structure which can, if desired, contain an oxygen or sulphur atom or an -NH- or -N($C_1$-$C_4$-alkyl)- bridge as a member of the ring, with the exception of the compound 4-trifluoroacetyl-2,3-dihydro-1,4-benzothiazine.

15. Compounds according to claim 14, characterised in that X is oxygen.

16. Compounds according to claim 14, characterised in that $R^1$ is $C_2$-$C_6$-haloalkenyl, or $C_1$-$C_6$-alkyl which is substituted by at least two halogen atoms.

17. Compounds according to claim 14, characterised in that $R^2$ and $R^5$ are hydrogen.

18. Compounds according to claim 14, characterised in that $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl.

19. Compounds according to claim 14, characterised in that $R^6$ and $R^7$ independently of one another are hydrogen or $C_1$-$C_4$-alkyl.

20. Compounds according to claim 18, characterised in that $R^3$ and $R^4$ independently of one another are hydrogen or methyl.

21. Compounds according to claim 16, characterised in that $R^1$ is $C_1$-$C_4$-alkyl which is substituted by at least two halogen atoms.

22. Compounds according to claim 21, characterised in that $R^1$ is dichloromethyl.

23. Compounds according to claim 14, characterised in that X is oxygen, $R^1$ is $C_2$-$C_6$-haloalkyl, or $C_1$-$C_4$-alkyl

which is substituted by at least two halogen atoms, $R^2$ and $R^5$ are hydrogen, $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl and $R^6$ and $R^7$ independently of one another are hydrogen or $C_1$-$C_4$-alkyl.

24. Compounds according to claim 23, characterised in that $R^1$ is $C_1$-$C_4$-alkyl which is substituted by at least two halogen atoms, and $R^3$, $R^4$, $R^6$ and $R^7$ independently of one another are hydrogen or methyl.

25. Compounds according to claim 24, characterised in that $R^1$ is dichloromethyl.

26. 4-Dichloroacetyl-2,3-dihydro-3-methyl-1,4-benzoxazine according to claim 14.

27. 4-Dichloroacetyl-2,3-dihydro-3,6-dimethyl-1,4-benzoxazine according to claim 14.

28. A process for selectively controlling weeds in crops of useful plants, characterised in that the cultivated plants or the area on which they are cultivated is treated with both a herbicide and an effective amount of an acylamide derivative of the formula I, according to claim 1, as safener.

29. The use of the acylamide derivatives of the formula I according to claim 1 for protecting cultivated plants against the harmful action of herbicides.

30. A process for protecting cultivated plants against damage occurring on the application of herbicides, characterised in that either the area on which the plants are cultivated is treated before or during the application of the herbicide, or the seed or cuttings of the plants or the plant itself is/are treated, with an effective amount of an acylamide derivative of the formula I according to claim 1.

31. Seed of useful plants which has been treated with an amount having an antagonistic action of an acylamide derivative of the formula I according to claim 1.

32. A process for the production of compounds of the formula I, in which $R^1$ is $C_1$-$C_6$-cyanoalkyl, $C_2$-$C_6$-haloalkenyl, or $C_1$-$C_6$-haloalkyl which is substituted by at least two halogen atoms, according to claim 14, characterised in that an acyl halide of the formula II

$R^1$ - CO - Hal (II)

in which $R^1$ is $C_1$-$C_6$-cyanoalkyl, $C_2$-$C_6$-haloalkenyl, or $C_1$-$C_6$-alkyl which is substituted by at least two halogen atoms, and Hal is chlorine or bromine, is reacted in the presence of an acid-binding agent with an amine of the formula III

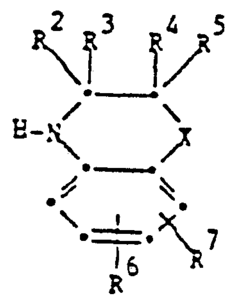

(III)

in which $R^2$, $R^3$, $R^4$, $R^5$, $R^6$, $R^7$ and X are as defined under formula I, with the proviso that $R^1$ may not represent trifluoromethyl when, simultaneously, $R^2$, $R^3$, $R^4$, $R^5$, $R^6$ and $R^7$ represent hydrogen and X represents sulphur.

**Claims** for the contracting state: AT

1. A composition for protecting cultivated plants against the phytotoxic action of herbicides, characterised in that, in addition to inert carrier material and, if desired, the herbicide, it contains, as the antagonising active substance, an acylamide derivative of the formula I

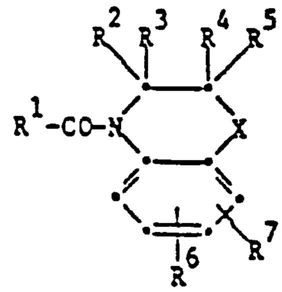

(I)

in which

X is oxygen, sulphur, -SO- or $-SO_2-$,

$R^1$ is $C_1$-$C_6$-haloalkyl, $C_1$-$C_6$-cyanoalkyl or $C_2$-$C_6$-haloalkenyl,

$R^2$ and $R^5$ independently of one another are hydrogen, $C_1$-$C_4$-alkyl, $C_1$-$C_4$-haloalkyl, $C_2$-$C_4$-alkenyl, $C_2$-$C_4$-alkynyl or $C_2$-$C_4$-alkoxyalkyl,

$R^3$ and $R^4$ independently of one another are hydrogen, $C_2$-$C_4$-alkenyl, $C_2$-$C_4$-alkynyl, cyano, $C_2$-$C_4$-alkoxyalkyl, $-COOR^8$, $-CO-NR^9R^{10}$, or $C_1$-$C_4$-alkyl which is unsubstituted or substituted by halogen, cyano or by -CO-A,

$R^6$ and $R^7$ independently of one another are hydrogen, halogen, $C_1$-$C_4$-alkyl, $C_1$-$C_4$-alkoxy, $C_1$-$C_4$-haloalkyl or $C_1$-$C_4$-haloalkoxy,

A is $C_1$-$C_4$-alkyl, $C_2$-$C_4$-alkenyl, $C_1$-$C_4$-alkoxy, $C_3$-$C_4$-alkenyloxy, $C_3$-$C_4$-alkynyloxy or $-NR^{11}R^{12}$,

$R^8$, $R^{10}$ and $R^{12}$ independently of one another are hydrogen, $C_1$-$C_4$-alkyl, $C_3$-$C_4$-alkenyl, $C_3$-$C_4$-alkynyl or $C_3$-$C_4$-alkoxyalkyl, and

$R^9$ and $R^{11}$ independently of one another are hydrogen, $C_1$-$C_4$-alkyl, $C_3$-$C_4$-alkenyl, $C_1$-$C_4$-alkoxy, $C_3$-$C_4$-alkynyl or $C_3$-$C_4$-alkoxyalkyl,

it being also possible for $R^9$ and $R^{10}$ and also $R^{11}$ and $R^{12}$, together with the nitrogen atom linking them, to form a 5-membered or 6-membered saturated heterocyclic structure which can, if desired, contain an oxygen or sulphur atom or an -NH- or $-N(C_1$-$C_4$-alkyl)- bridge as a member of the ring.

2. A composition according to claim 1, characterised in that X is oxygen.

3. A composition according to claim 1, characterised in that $R^1$ is $C_1$-$C_6$-haloalkyl or $C_2$-$C_6$-haloalkenyl.

4. A composition according to claim 1, characterised in that $R^2$ and $R^5$ are hydrogen.

5. A composition according to claim 1, characterised in that $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl.

6. A composition according to claim 1, characterised in that $R^6$ and $R^7$ independently of one another are hydrogen or $C_1$-$C_4$-alkyl.

7. A composition according to claim 5, characterised in that $R^3$ and $R^4$ independently of one another are hydrogen or methyl.

8. A composition according to claim 3, characterised in that $R^1$ is $C_1$-$C_4$-haloalkyl.

9. A composition according to claim 3, characterised in that $R^1$ is dichloromethyl.

10. A composition according to claim 1, characterised in that X is oxygen, $R^1$ is $C_1$-$C_6$-haloalkyl or $C_2$-$C_6$-haloalkenyl, $R^2$ and $R^5$ are hydrogen, $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl and $R^6$ and $R^7$ independently of one another are hydrogen or $C_1$-$C_4$-alkyl.

11. A composition according to claim 9, characterised in that $R^1$ is $C_1$-$C_4$-haloalkyl and $R^3$, $R^4$, $R^6$ and $R^7$ independently of one another are hydrogen or methyl.

12. A composition according to claim 11, characterised in that $R^1$ is dichloromethyl.

13. A composition according to claim 1, characterised in that it contains as the antagonising active substance, 4-dichloroacetyl-2,3-dihydro-3-methyl-1,4-benzoxazine, 4-dichloroacetyl-2,3-dihydro-3,6-dimethyl-1,4-benzoxazine or 4-(2-chloropropionyl)-2,3-dihydro-3-methyl-1,4-benzoxazine.

14. A process for the preparation of the acylamide derivatives of the formula I

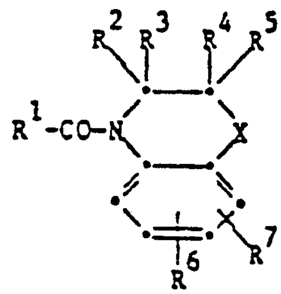

(I)

in which

X is oxygen, sulphur, -SO- or $-SO_2-$,

$R^1$ is $C_1$-$C_6$-cyanoalkyl, $C_2$-$C_6$-haloalkenyl, or $C_1$-$C_6$-alkyl which is substituted by at least two halogen atoms,

$R^2$ and $R^5$ independently of one another are hydrogen, $C_1$-$C_4$-alkyl, $C_1$-$C_4$-haloalkyl, $C_2$-$C_4$-alkenyl, $C_2$-$C_4$-alkynyl or $C_2$-$C_4$-alkoxyalkyl,

$R^3$ and $R^4$ independently of one another are hydrogen, $C_2$-$C_4$-alkenyl, $C_2$-$C_4$-alkynyl, cyano, $C_2$-$C_4$-alkoxyalkyl, $-COOR^8$, $-CO-NR^9R^{10}$, or $C_1$-$C_4$-alkyl which is unsubstituted or substituted by halogen, cyano or by -CO-A,

$R^6$ and $R^7$ independently of one another are hydrogen, halogen, $C_1$-$C_4$-alkyl, $C_1$-$C_4$-alkoxy, $C_1$-$C_4$-haloalkyl or $C_1$-$C_4$-haloalkoxy,

A is $C_1$-$C_4$-alkyl, $C_2$-$C_4$-alkenyl, $C_1$-$C_4$-alkoxy, $C_3$-$C_4$-alkenyloxy, $C_3$-$C_4$-alkynyloxy or $-NR^{11}R^{12}$,

$R^8$, $R^{10}$ and $R^{12}$ independently of one another are hydrogen, $C_1$-$C_4$-alkyl, $C_3$-$C_4$-alkenyl, $C_3$-$C_4$-alkynyl or $C_3$-$C_4$-alkoxyalkyl, and

$R^9$ and $R^{11}$ independently of one another are hydrogen, $C_1$-$C_4$-alkyl, $C_3$-$C_4$-alkenyl, $C_1$-$C_4$-alkoxy, $C_3$-$C_4$-alkynyl or $C_3$-$C_4$-alkoxyalkyl,

it being also possible for $R^9$ and $R^{10}$ and also $R^{11}$ and $R^{12}$, together with the nitrogen atom linking them, to form a 5-membered or 6-membered saturated heterocyclic structure which can, if desired, contain an oxygen or sulphur atom or an -NH- or $-N(C_1$-$C_4$-alkyl)- bridge as a member of the ring, with the exception of the compound 4-trifluoroacetyl-2,3-dihydro-1,4-benzothiazine, characterised in that an acyl halide of the formula II

$R^1$ - CO - Hal (II)

in which $R^1$ is $C_1$-$C_6$-cyanoalkyl, $C_2$-$C_6$-haloalkenyl, or $C_1$-$C_6$-alkyl which is substituted by at least two halogen atoms, and Hal is chlorine or bromine, is reacted in the presence of an acid-binding agent with an amine of the formula III

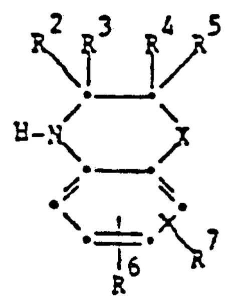

(III)

in which $R^2$, $R^3$, $R^4$, $R^5$, $R^6$, $R^7$ and X are as defined under formula I, with the proviso that $R^1$ may not represent trifluoromethyl when, simultaneously, $R^2$, $R^3$, $R^4$, $R^5$, $R^6$ and $R^7$ represent hydrogen and X represents sulphur.

15. A process according to claim 14, characterised in that X is oxygen.

16. A process according to claim 14, characterised in that $R^1$ is $C_2$-$C_6$-haloalkenyl, or $C_1$-$C_6$-alkyl which is substituted by at least two halogen atoms.

17. A process according to claim 14, characterised in that $R^2$ and $R^5$ are hydrogen.

18. A process according to claim 14, characterised in that $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl.

19. A process according to claim 14, characterised in that $R^6$ and $R^7$ independently of one another are hydrogen or $C_1$-$C_4$-alkyl.

20. A process according to claim 18, characterised in that $R^3$ and $R^4$ independently of one another are hydrogen or methyl.

21. A process according to claim 16, characterised in that $R^1$ is $C_1$-$C_4$-alkyl which is substituted by at least

two halogen atoms.

22. A process according to claim 21, characterised in that $R^1$ is dichloromethyl.

23. A process according to claim 14, characterised in that X is oxygen, $R^1$ is $C_2$-$C_6$-haloalkyl, or $C_1$-$C_4$-alkyl which is substituted by at least two halogen atoms, $R^2$ and $R^5$ are hydrogen, $R^3$ and $R^4$ independently of one another are hydrogen, $C_1$-$C_4$-alkyl, $C_1$-$C_4$-alkoxycarbonylmethyl or $C_1$-$C_4$-alkoxycarbonyl and $R^6$ and $R^7$ independently of one another are hydrogen or $C_1$-$C_4$-alkyl.

24. Compounds according to claim 23, characterised in that $R^1$ is $C_1$-$C_4$-alkyl which is substituted by at least two halogen atoms, and $R^3$, $R^4$, $R^6$ and $R^7$ independently of one another are hydrogen or methyl.

25. A process according to claim 24, characterised in that $R^1$ is dichloromethyl.

26. A process according to claim 14, characterised in that 4-dichloroacetyl-2,3-dihydro-3-methyl-1,4-benzoxazine is prepared.

27. A process according to claim 14, characterised in that 4-dichloroacetyl-2,3-dihydro-3,6-dimethyl-1,4-benzoxazine is prepared.

28. A process for selectively controlling weeds in crops of useful plants, characterised in that the cultivated plants or the area on which they are cultivated is treated with both a herbicide and an effective amount of an acylamide derivative of the formula I, according to claim 1, as safener.

29. The use of the acylamide derivatives of the formula I according to claim 1 for protecting cultivated plants against the harmful action of herbicides.

30. A process for protecting cultivated plants against damage occurring on the application of herbicides, characterised in that either the area on which the plants are cultivated is treated before or during the application of the herbicide, or the seed or cuttings of the plants or the plant itself is/are treated, with an effective amount of an acylamide derivative of the formula I according to claim 1.

**Revendications** pour les états contractants BE, CH, LI, DE, FR, IT, NL, GB

1. Un produit pour protéger les végétaux cultivés contre les effets de phytotoxicité des herbicides, caractérisé en ce qu'il contient en tant que substance active antagonisante, avec un véhicule inerte et le cas échéant l'herbicide, un dérivé d'acylamide de formule I

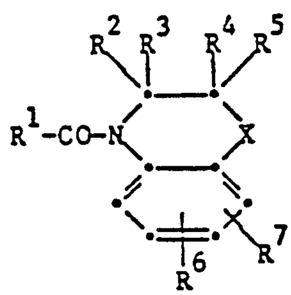

(I),

dans laquelle

X représente l'oxygène, le soufre, -SO- ou -$SO_2$-,

$R^1$ représente un groupe halogénoalkyle en $C_1$-$C_6$, cyanalkyle en $C_1$-$C_6$ ou halogénoalcényle en $C_2$-$C_6$,

$R^2$ et $R^5$ représentent chacun, indépendamment l'un de l'autre, l'hydrogène, un groupe alkyle en $C_1$-$C_4$, halogénoalkyle en $C_1$-$C_4$, alcényle en $C_2$-$C_4$, alcynyle en $C_2$-$C_4$ ou alcoxyalkyle en $C_2$-$C_4$,

$R^3$ et $R^4$ représentent chacun, indépendamment l'un de l'autre, l'hydrogène, un groupe alcényle en $C_2$-$C_4$, alcynyle en $C_2$-$C_4$, cyano, alcoxyalkyle en $C_2$-$C_4$, -$COOR^8$, -CO-$NR^9R^{10}$ ou un groupe alkyle en $C_1$-$C_4$ non substitué ou substitué par des halogènes, des groupes cyano ou -CO-A,

$R^6$ et $R^7$ représentent chacun, indépendamment l'un de l'autre, l'hydrogène, un halogène, un groupe alkyle en $C_1$-$C_4$, alcoxy en $C_1$-$C_4$, halogénoalkyle en $C_1$-$C_4$ ou halogénoalcoxy en $C_1$-$C_4$,

A représente un groupe alkyle en $C_1$-$C_4$, alcényle en $C_2$-$C_4$, alcoxy en $C_1$-$C_4$, alcényloxy en $C_3$-$C_4$, alcynyloxy en $C_3$-$C_4$ ou -$NR^{11}R^{12}$,

$R^8$ $R^{10}$ et $R^{12}$ représentent chacun, indépendamment les uns des autres l'hydrogène un groupe alkyle en $C_1$-$C_4$, alcényle en $C_3$-$C_4$, alcynyle en $C_3$-$C_4$ ou alcoxyalkyle en $C_3$-$C_4$, et

$R^9$ et $R^{11}$ représentent chacun, indépendamment l'un de l'autre, l'hydrogène, un groupe alkyle en $C_1$-$C_4$, alcényle en $C_3$-$C_4$, alcoxy en $C_1$-$C_4$, alcynyle en $C_3$-$C_4$ ou alcoxyalkyle en $C_3$-$C_4$,

$R^9$ et $R^{10}$, d'une part, et $R^{11}$ et $R^{12}$, d'autre part, pouvant également former ensemble et avec l'atome d'azote auquel ils sont reliés, un hétérocycle saturé à 5 ou 6 chaînons qui peut contenir le cas échéant en tant que chaînon cyclique un atome d'oxygène ou de soufre ou un pont -NH- ou -N(alkyle en $C_1$-$C_4$)-.

2. Produit selon la revendication 1, caractérisé en ce que X représente l'oxygène.

3. Produit selon la revendication 1, caractérisé en ce que $R^1$ représente un groupe halogénoalkyle en $C_1$-$C_6$ ou

halogénoalcényle en $C_2$-$C_6$.

4. Produit selon la revendication 1, caractérisé en ce que $R^2$ et $R^5$ représentent l'hydrogène.

5. Produit selon la revendication 1, caractérisé en ce que $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-carbonyle.

6. Produit selon la revendication 1, caractérisé en ce que $R^6$ et $R^7$ représentent chacun, indépendamment l'un de l'autre, l'hydrogène ou un groupe alkyle en $C_1$-$C_4$.

7. Produit selon la revendication 5, caractérisé en ce que $R^3$ et $R^4$ représentent chacun, indépendamment l'un de l'autre, l'hydrogène ou un groupe méthyle.

8. Produit selon la revendication 3, caractérisé en ce que $R^1$ représente un groupe halogénoalkyle en $C_1$-$C_4$.

9. Produit selon la revendication 3, caractérisé en ce que $R^1$ représente un groupe dichlorométhyle.

10. Produit selon la revendication 1, caractérisé en ce que X représente l'oxygène, $R^1$ représente un groupe halogénoalkyle en $C_1$-$C_6$ ou halogénoalcényle en $C_2$-$C_6$, $R^2$ et $R^5$ représentent l'hydrogène, $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-carbonyle et $R^6$ et $R^7$ représentent chacun, indépendamment l'un de l'autre, l'hydrogène ou un groupe alkyle en $C_1$-$C_4$.

11. Produit selon la revendication 9, caractérisé en ce que $R^1$ représente un groupe halogénoalkyle en $C_1$-$C_4$ et $R^3$, $R^4$, $R^6$ et $R^7$ représentent chacun, indépendamment les uns des autres, l'hydrogène ou un groupe méthyle.

12. Produit selon la revendication 11, caractérisé en ce que $R^1$ représente un groupe dichlorométhyle.

13. Produit selon la revendication 1, caractérisé en ce qu'il contient, en tant que substance active antagonisante la 4-dichloracétyl-2,3-dihydro-3-méthyl-1,4-benzoxazine, la 4-dichloracétyl-2,3-dihydro-3,6-diméthyl-1,4-benzoxazine ou la 4-(2-chloropropionyl)-2,3-dihydro-3-méthyl-1,4-benzoxazine.

14. Nouveaux dérivés d'acylamides de formule I

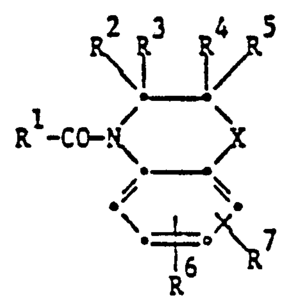

(I),

dans laquelle

X représente l'oxygène, le soufre, -SO- ou -$SO_2$-,

$R^1$ représente un groupe cyanalkyle en $C_1$-$C_6$, halogénoalcényle en $C_2$-$C_6$ ou alkyle en $C_1$-$C_6$ substitue par au moins deux atomes d'halogènes,

$R^2$ et $R^5$ représentent chacun, indépendamment l'un de l'autre, l'hydrogène, un groupe alkyle en $C_1$-$C_4$, halogénoalkyle en $C_1$-$C_4$, alcényle en $C_2$-$C_4$, alcynyle en $C_2$-$C_4$ ou alcoxyalkyle en $C_2$-$C_4$,

$R^3$ et $R^4$ représentent chacun, indépendamment l'un de l'autre, l'hydrogène, un groupe alcényle en $C_2$-$C_4$, alcynyle en $C_2$-$C_4$, cyano, alcoxyalkyle en $C_2$-$C_4$, -$COOR^8$, -CO-$NR^9R^{10}$ ou un groupe alkyle en $C_1$-$C_4$ non substitué ou substitué par des halogènes, des groupes cyano ou -CO-A,

$R^6$ et $R^7$ représentent chacun, indépendamment l'un de l'autre, l'hydrogène, un halogène, un groupe alkyle en $C_1$-$C_4$, alcoxy en $C_1$-$C_4$, halogénoalkyle en $C_1$-$C_4$ ou halogénoalcoxy en $C_1$-$C_4$,

A représente un groupe alkyle en $C_1$-$C_4$ alcényle en $C_2$-$C_4$, alcoxy en $C_1$-$C_4$, alcényloxy en $C_3$-$C_4$, alcynyloxy en $C_3$-$C_4$ ou -$NR^{11}R^{12}$,

$R^8$, $R^{10}$ et $R^{12}$ représentent chacun, indépendamment les uns des autres, l'hydrogène, un groupe alkyle en $C_1$-$C_4$, alcényle en $C_3$-$C_4$ alcynyle en $C_3$-$C_4$ ou alcoxyalkyle en $C_3$-$C_4$, et

$R^9$ et $R^{11}$ représentent chacun, indépendamment l'un de l'autre, l'hydrogène, un groupe alkyle en $C_1$-$C_4$, alcényle en $C_3$-$C_4$, alcoxy en $C_1$-$C_4$, alcynyle en $C_3$-$C_4$ ou alcoxyalkyle en $C_3$-$C_4$,

$R^9$ et $R^{10}$, d'une part, et $R^{11}$ et $R^{12}$, d'autre part, pouvant également former ensemble et avec l'atome d'azote auquel ils sont reliés un hétérocycle saturé à 5 ou 6 chaînons, qui peut contenir le cas échéant en tant que chaînon cyclique un atome d'oxygène ou de soufre ou un pont -NH- ou -N(alkyle en $C_1$-$C_4$)- ; à l'exception du composé 4-trifluoracétyl-2,3-dihydro-1,4-benzothiazine.

15. Composés selon la revendication 14, caractérisés en ce que X représente l'oxygène.

16. Composés selon la revendication 14, caractérisés en ce que $R^1$ représente un groupe halogénoalcényle en $C_2$-$C_6$ ou un groupe alkyle en $C_1$-$C_6$ substitué par au moins deux atomes d'halogènes.

17. Composés selon la revendication 14, caractérisés en ce que $R^2$ et $R^5$ représentent l'hydrogène.

18. Composés selon la revendication 14, caractérisés en ce que $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou

(alcoxy en $C_1$-$C_4$)-carbonyle.

19. Composés selon la revendication 14, caractérisés en ce que $R^6$ et $R^7$ représentent chacun indépendamment l'un de l'autre, l'hydrogène ou un groupe alkyle en $C_1$-$C_4$.

20. Composés selon la revendication 18, caractérisés en ce que $R^3$ et $R^4$ représentent chacun, indépendamment l'un de l'autre, l'hydrogene ou un groupe méthyle.

21. Composés selon la revendication 16, caractérisés en ce que $R^1$ représente un groupe alkyle en $C_1$-$C_4$ substitué par au moins deux atomes d'halogènes.

22. Composés selon la revendication 21, caractérisés en ce que $R^1$ représente un groupe dichlorométhyle.

23. Composés selon la revendication 14, caractérisés en ce que X représente l'oxygène, $R^1$ represente un groupe halogénoalkyle en $C_2$-$C_6$ ou un groupe alkyle en $C_1$-$C_4$ substitué par au moins deux atomes d'halogènes, $R^2$ et $R^5$ représentent l'hydrogène, $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-carbonyle et $R^6$ et $R^7$ représentent chacun, indépendamment l'un de l'autre l'hydrogène ou un groupe alkyle en $C_1$-$C_4$.

24. Composés selon la revendication 23, caractérisés en ce que $R^1$ représente un groupe alkyle en $C_1$-$C_4$ substitué par au moins deux atomes d'halogènes et $R^3$, $R^4$, $R^6$ et $R^7$ représentent chacun, indépendamment les uns des autres, l'hydrogène ou un groupe méthyle.

25. Composés selon la revendication 24, caractérisés en ce que $R^1$ représente un groupe dichlorométhyle.

26. La 4-dichloracétyl-2,3-dihydro-3-méthyl-1,4-benzoxazine selon la revendication 14.

27. La 4-dichloracétyl-2,3-dihydro-3,6-diméthyl-1,4-benzoxazine selon la revendication 14.

28. Procédé pour combattre sélectivement les végétaux adventices dans les cultures de végétaux cultivés, caractérisé en ce que l'on traite les végétaux cultivés ou leur aire de culture à la fois par un herbicide et par une quantité efficace d'un dérivé d'acylamide de formule I selon la revendication 1 servant d'antidote.

29. Utilisation des dérivés d'acylamides de formule I selon la revendication 1, pour la protection des végétaux cultivés contre les dommages provoqués par les herbicides.

30. Procédé pour protéger les vegétaux cultivés contre les dommages résultant de l'application d'herbicides, caractérisé en ce que l'on traite l'aire de culture de la plante avant ou durant l'application de l'herbicide ou les semences ou les pousses de la plante ou la plante elle-même par une quantité efficace d'un dérivé d'acylamide de formule I selon la revendication 1.

31. Semences de végétaux utiles qui ont été traitées par une quantité efficace en tant qu'antagoniste d'un dérivé d'acylamide de formule I selon la revendication 1.

32. Procédé de préparation des composés de formule I dans laquelle $R^1$ représente un groupe cyanalkyle en $C_1$-$C_6$, halogénoalcényle en $C_2$-$C_6$ ou un groupe halogénoalkyle en $C_1$-$C_6$ substitué par au moins deux atomes d'halogènes, selon la revendication 14, caractérisé en ce que l'on fait réagir un halogénure d'acyle de formule II

$$R^1 - CO - Hal \qquad (II)$$

dans laquelle $R^1$ représente un groupe cyanalkyle en $C_1$-$C_6$, halogénoalcényle en $C_2$-$C_6$ ou un groupe alkyle en $C_1$-$C_6$ substitué par au moins deux atomes d'halogènes et Hal représente le chlore ou le brome, en présence d'un capteur d'acide, avec une amine de formule III

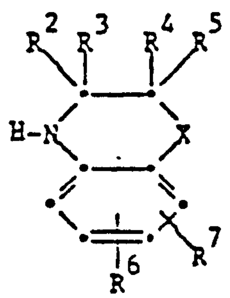

(III),

dans laquelle $R^2$ $R^3$ $R^4$ $R^5$ $R^6$ $R^7$ et X ont les significations indiquées en référence à la formule I, étant spécifié que $R^1$ ne peut représenter un groupe trifluorométhyle lorsque, en même temps, $R^2$, $R^3$, $R^4$, $R^5$, $R^6$ et $R^7$ représentent l'hydrogène et X le soufre.

**Revendications** pour l'état contractant: AT

1. Un produit pour protéger les végétaux cultivés contre les effets de phytotoxicité des herbicides, caractérisé en ce qu'il contient en tant que substance active antagonisante, avec un véhicule inerte et le cas échéant l'herbicide, un dérivé d'acylamide de formule I

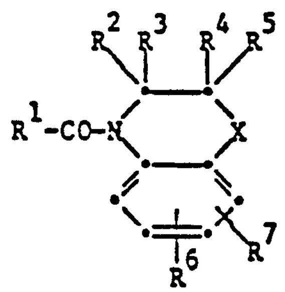

(I),

dans laquelle

X représente l'oxygène, le soufre, -SO- ou $-SO_2-$,

$R^1$ représente un groupe halogénoalkyle en $C_1$-$C_6$, cyanalkyle en $C_1$-$C_6$ ou halogénoalcényle en $C_2$-$C_6$,

$R^2$ et $R^5$ représentent chacun, indépendamment l'un de l'autre, l'hydrogène, un groupe alkyle en $C_1$-$C_4$, halogénoalkyle en $C_1$-$C_4$, alcényle en $C_2$-$C_4$, alcynyle en $C_2$-$C_4$ ou alcoxyalkyle en $C_2$-$C_4$,

$R^3$ et $R^4$ représentent chacun, indépendamment l'un de l'autre, l'hydrogène, un groupe alcényle en $C_2$-$C_4$, alcynyle en $C_2$-$C_4$, cyano, alcoxyalkyle en $C_2$-$C_4$, $-COOR^8$, $-CO-NR^9R^{10}$ ou un groupe alkyle en $C_1$-$C_4$ non substitué ou substitué par des halogènes, des groupes cyano ou -CO-A,

$R^6$ et $R^7$ représentent chacun, indépendamment l'un de l'autre, l'hydrogène, un halogène, un groupe alkyle en $C_1$-$C_4$, alcoxy en $C_1$-$C_4$, halogénoalkyle en $C_1$-$C_4$ ou halogénoalcoxy en $C_1$-$C_4$,

A représente un groupe alkyle en $C_1$-$C_4$, alcényle en $C_2$-$C_4$, alcoxy en $C_1$-$C_4$, alcényloxy en $C_3$-$C_4$, alcynyloxy en $C_3$-$C_4$ ou $-NR^{11}R^{12}$,

$R^8$ $R^{10}$ et $R^{12}$ représentent chacun indépendamment les uns des autres l'hydrogène un groupe alkyle en $C_1$-$C_4$, alcényle en $C_3$-$C_4$, alcynyle en $C_3$-$C_4$ ou alcoxyalkyle en $C_3$-$C_4$, et

$R^9$ et $R^{11}$ représentent chacun, indépendamment l'un de l'autre l'hydrogène, un groupe alkyle en $C_1$-$C_4$, alcényle en $C_3$-$C_4$, alcoxy en $C_1$-$C_4$, alcynyle en $C_3$-$C_4$ ou alcoxyalkyle en $C_3$-$C_4$,

$R^9$ et $R^{10}$ d'une part, et $R^{11}$ et $R^{12}$, d'autre part, pouvant également former ensemble et avec l'atome d'azote auquel ils sont reliés, un hétérocycle saturé à 5 ou 6 chaînons qui peut contenir le cas échéant en tant que chaînon cyclique un atome d'oxygène ou de soufre ou un pont -NH- ou -N(alkyle en $C_1$-$C_4$).

2. Produit selon la revendication 1, caractérisé en ce que X représente l'oxygène.

3. Produit selon la revendication 1, caractérisé en ce que $R^1$ représente un groupe halogénoalkyle en $C_1$-$C_6$ ou halogénoalcényle en $C_2$-$C_6$.

4. Produit selon la revendication 1, caractérisé en ce que $R^2$ et $R^5$ représentent l'hydrogène.

5. Produit selon la revendication 1, caractérisé en ce que $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-carbonyle.

6. Produit selon la revendication 1, caractérisé en ce que $R^6$ et $R^7$ représentent chacun, indépendamment l'un de l'autre, l'hydrogène ou un groupe alkyle en $C_1$-$C_4$.

7. Produit selon la revendication 5, caractérisé en ce que $R^3$ et $R^4$ représentent chacun, indépendamment l'un de l'autre, l'hydrogène ou un groupe méthyle.

8. Produit selon la revendication 3, caractérisé en ce que $R^1$ représente un groupe halogénoalkyle en $C_1$-$C_4$.

9. Produit selon la revendication 3, caractérisé en ce que $R^1$ représente un groupe dichlorométhyle.

10. Produit selon la revendication 1, caractérisé en ce que X représente l'oxygène, $R^1$ représente un groupe halogénoalkyle en $C_1$-$C_6$ ou halogénoalcényle en $C_2$-$C_6$, $R^2$ et $R^5$ représentent l'hydrogène, $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-carbonyle et $R^6$ et $R^7$ représentent chacun, indépendamment l'un de l'autre, l'hydrogène ou un groupe alkyle en $C_1$-$C_4$.

11. Produit selon la revendication 9, caractérisé en ce que $R^1$ représente un groupe halogénoalkyle en $C_1$-$C_4$ et $R^3$, $R^4$, $R^6$ et $R^7$ représentent chacun, indépendamment les uns des autres, l'hydrogène ou un groupe méthyle.

12. Produit selon la revendication 11, caractérisé en ce que $R^1$ représente un groupe dichlorométhyle.

13. Produit selon la revendication 1, caractérisé en ce qu'il contient, en tant que substance active antagonisante la 4-dichloracétyl-2,3-dihydro-3-méthyl-1,4-benzoxazine, la 4-dichloracétyl-2,3-dihydro-3,6-diméthyl-1,4-benzoxazine ou la 4-(2-chloropropionyl)-2,3-dihydro-3-méthyl-1,4-benzoxazine.

14. Procédé de préparation des dérivés d'acylamides de formule I

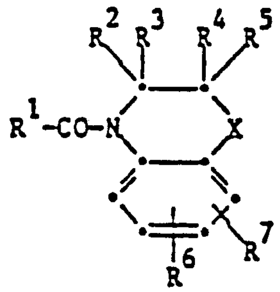

(I),

dans laquelle

X représente l'oxygène le soufre, -SO- ou $-SO_2-$,

$R^1$ représente un groupe cyanalkyle en $C_1$-$C_6$, halogénoalcényle en $C_2$-$C_6$ ou un groupe alkyle en $C_1$-$C_6$ substitué par au moins deux atomes d'halogènes,

$R^2$ et $R^5$ représentent chacun, indépendamment l'un de l'autre, l'hydrogène, un groupe alkyle en $C_1$-$C_4$, halogénoalkyle en $C_1$-$C_4$, alcényle en $C_2$-$C_4$, alcynyle en $C_2$-$C_4$ ou alcoxyalkyle en $C_2$-$C_4$,

$R^3$ et $R^4$ représentent chacun, indépendamment l'un de l'autre, l'hydrogène, un groupe alcényle en $C_2$-$C_4$, alcynyle en $C_2$-$C_4$, cyano, alcoxyalkyle en $C_2$-$C_4$, $-COOR^8$ $-CO-NR^9R^{10}$ ou un groupe alkyle non substitué ou substitué par des halogènes, des groupes cyano ou -CO-A,

$R^6$ et $R^7$ représentent chacun, indépendamment l'un de l'autre, l'hydrogène, un halogène, un groupe alkyle en $C_1$-$C_4$, alcoxy en $C_1$-$C_4$, halogénoalkyle en $C_1$-$C_4$ ou halogénoalcoxy en $C_1$-$C_4$,

A représente un groupe alkyle en $C_1$-$C_4$, alcényle en $C_2$-$C_4$, alcoxy en $C_1$-$C_4$, alcényloxy en $C_3$-$C_4$, alcynyloxy en $C_3$-$C_4$ ou $-NR^{11}R^{12}$,

$R^8$, $R^{10}$ et $R^{12}$ représentent chacun, indépendamment les uns des autres, l'hydrogène, un groupe alkyle en $C_1$-$C_4$, alcényle en $C_3$-$C_4$, alcynyle en $C_3$-$C_4$ ou alcoxyalkyle en $C_3$-$C_4$, et

$R^9$ et $R^{11}$ représentent chacun, indépendamment l'un de l'autre, l'hydrogène, un groupe alkyle en $C_1$-$C_4$, alcényle en $C_3$-$C_4$, alcoxy en $C_1$-$C_4$, alcynyle en $C_3$-$C_4$ ou alcoxyalkyle en $C_3$-$C_4$,

$R^9$ et $R^{10}$ d'une part, et $R^{11}$ et $R^{12}$ d'autre part, pouvant également former avec l'atome d'azote auquel ils sont reliés un hétérocycle saturé à 5 ou 6 chaînons qui peut contenir le cas échéant en tant que chaînon cyclique un atome d'oxygène ou de soufre ou un pont -NH- ou -N(alkyle en $C_1$-$C_4$)-, à l'exception du composé 4-trifluoracétyl-2,3-dihydro-1,4-benzothiazine caractérisé en ce que l'on fait réagir un halogénure d'acyle de formule II

$R^1$ - CO - Hal (II),

dans laquelle $R^1$ représente un groupe cyanalkyle en $C_1$-$C_6$, halogénoalcényle en $C_2$-$C_6$ ou un groupe alkyle en $C_1$-$C_6$ substitué par au moins deux atomes d'halogènes, et Hal représente le chlore ou le brome, en présence d'un capteur d'acide, avec une amine de formule III

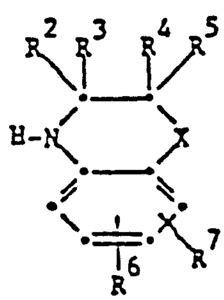

(III),

dans laquelle $R^2$ $R^3$ $R^4$, $R^5$, $R^6$, $R^7$ et X ont les significations indiquées en référence à la formule I, étant spécifié que $R^1$ ne peut représenter un groupe trifluoromethyle lorsque, en meme temps, $R^2$, $R^3$, $R^4$, $R^5$, $R^6$ et $R^7$ représentent l'hydrogène et X le soufre.

15. Procédé selon la revendication 14, caractérisé en ce que X représente l'oxygène.

16. Procédé selon la revendication 14, caractérisé en ce que $R^1$ représente un groupe halogénoalcényle en $C_2$-$C_6$ ou un groupe alkyle en $C_1$-$C_6$ substitué par au moins deux atomes d'halogènes.

17. Procédé selon la revendication 14, caractérisé en ce que $R^2$ et $R^5$ représentent l'hydrogène.

18. Procédé selon la revendication 14, caractérisé en ce que $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-

carbonyle.

19. Procédé selon la revendication 14, caractérisé en ce que $R^6$ et $R^7$ représentent chacun, indépendamment l'un de l'autre, l'hydrogène ou un groupe alkyle en $C_1$-$C_4$.

20. Procédé selon la revendication 18, caractérisé en ce que $R^3$ et $R^4$ représentent chacun, indépendamment l'un de l'autre, l'hydrogène ou un groupe méthyle.

21. Procédé selon la revendication 16, caractérisé en ce que $R^1$ représente un groupe alkyle en $C_1$-$C_4$ substitué par au moins deux atomes d'halogènes.

22. Procédé selon la revendication 21, caractérisé en ce que $R^1$ représente un groupe dichlorométhyle.

23. Procédé selon la revendication 14, caractérisé en ce que X représente l'oxygène, $R^1$ représente un groupe halogénoalkyle en $C_2$-$C_6$ ou un groupe alkyle en $C_1$-$C_4$ substitué par au moins deux atomes d'halogènes, $R^2$ et $R^5$ représentent l'hydrogène, $R^3$ et $R^4$ représentent chacun, indépendamment l'un de l'autre, l'hydrogène, un groupe alkyle en $C_1$-$C_4$, (alcoxy en $C_1$-$C_4$)-carbonylméthyle ou (alcoxy en $C_1$-$C_4$)-carbonyle et $R^6$ et $R^7$ représentent chacun, indépendamment l'un de l'autre, l'hydrogène ou un groupe alkyle en $C_1$-$C_4$.

24. Composés selon la revendication 23, caractérisés en ce que $R^1$ représente un groupe alkyle en $C_1$-$C_4$ substitué par au moins deux atomes d'halogènes et $R^3$, $R^4$, $R^6$ et $R^7$ représentent chacun, indépendamment les uns des autres, l'hydrogène ou un groupe méthyle.

25. Procédé selon la revendication 24, caractérisé en ce que $R^1$ représente un groupe dichlorométhyle.

26. Procédé selon la revendication 14, caractérisé en ce que l'on prépare la 4-dichloracétyl-2,3-dihydro-3-méthyl-1,4-benzoxazine.

27. Procédé selon la revendication 14, caractérisé en ce que l'on prépare la 4-dichloracétyl-2,3-dihydro-3,6-diméthyl-1,4-benzoxazine.

28. Procédé pour combattre sélectivement les vegétaux adventices dans les cultures de végétaux utiles, caractérisé en ce que l'on traite les végétaux cultivés ou leur aire de culture à la fois par un herbicide et par une quantité efficace d'un dérivé d'acylamide de formule I selon la revendication 1 servant d'antidote.

29. Utilisation des dérivés d'acylamides de formule I selon la revendication 1, pour la protection des végétaux cultivés contre les effets nocifs des herbicides.

30. Procédé pour protéger les végétaux cultivés contre les dommages résultant de l'application d'herbicides, caractérisé en ce que l'on traite l'aire de culture de la plante avant ou durant l'application de l'herbicide ou les semences ou les pousses de la plante ou la plante elle-même par une quantité efficace d'un dérivé d'acylamide de formule I selon la revendication 1.